(19) Europäisches Patentamt / European Patent Office / Office européen des brevets

(11) **EP 4 142 734 B1**

(12) **EUROPEAN PATENT SPECIFICATION**


(45) Date of publication and mention of the grant of the patent:
**20.08.2025 Bulletin 2025/34**

(21) Application number: **21727630.2**

(22) Date of filing: **27.04.2021**

(51) International Patent Classification (IPC):
***A61K 31/55*** *(2006.01)* ***A61K 9/00*** *(2006.01)*
***A61K 31/506*** *(2006.01)* ***A61K 33/34*** *(2006.01)*
***A61P 31/14*** *(2006.01)*

(52) Cooperative Patent Classification (CPC):
(C-Sets available)
**A61K 31/55; A61K 31/506; A61K 33/34; A61K 45/06; A61P 31/14;** A61K 9/0043 (Cont.)

(86) International application number:
**PCT/US2021/029375**

(87) International publication number:
**WO 2021/222230 (04.11.2021 Gazette 2021/44)**


(54) **LOCAL ADMINISTRATION OF NICOTINIC ACETYLCHOLINE RECEPTOR AGONISTS FOR THE INHIBITION OF CORONAVIRUS INFECTIONS**

LOKALE VERABREICHUNG VON NIKOTINISCHEN ACETYLCHOLINREZEPTORAGONISTEN ZUR HEMMUNG VON CORONAVIRUSINFEKTIONEN

ADMINISTRATION LOCALE D’AGONISTES DU RÉCEPTEUR NICOTINIQUE DE L’ACÉTYLCHOLINE POUR L’INHIBITION D’INFECTIONS À CORONAVIRUS


(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.04.2020 US 202063016886 P**

(43) Date of publication of application:
**08.03.2023 Bulletin 2023/10**



(73) Proprietor: **Oyster Point Pharma, Inc.**
**Princeton, New Jersey 08540 (US)**

(72) Inventors:
- **NAU, Jeffrey Alan**
  **Princeton, New Jersey 08540 (US)**
- **CARLSON, Eric C**
  **Burleson, Texas 76028 (US)**

(74) Representative: **Gill Jennings & Every LLP**
**The Broadgate Tower**
**20 Primrose Street**
**London EC2A 2ES (GB)**



(56) References cited:
**WO-A1-2016/064759 WO-A1-2017/023700**
**WO-A1-2021/016049 US-A1- 2020 281 972**
**US-B1- 6 261 589**

- **CHANGEUX JEAN-PIERRE ET AL: "A nicotinic hypothesis for Covid-19 with preventive and therapeutic implications", QEIOS, 21 April 2020 (2020-04-21), XP055814171, Retrieved from the Internet <URL:https://www.qeios.com/read/FXGQSB/pdf> DOI: 10.32388/FXGQSB**
- **KONSTANTINOS FARSALINOS ET AL: "Smoking, vaping and hospitalization for COVID-19", QEIOS, 3 April 2020 (2020-04-03), XP055731775, DOI: 10.32388/Z69O8A.11**
- **J L SAGRIPANTI ET AL: "Virus inactivation by copper or iron ions alone and in the presence of peroxide", APPLIED AND ENVIRONMENTAL MICROBIOLOGY, 1 December 1993 (1993-12-01), UNITED STATES, pages 4374 - 4376, XP055265467, Retrieved from the Internet <URL:http://www.ncbi.nlm.nih.gov/pmc/articles/PMC195916/pdf/aem00041-0406.pdf> [retrieved on 20160414]**



Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **SHEN ET AL: "Computational note on the SOD-like antioxidant potential of nicotine-copper(II) complexes", JOURNAL OF MOLECULAR STRUCTURE (THEOCHEM), ELSEVIER SCIENCE PUBLISHERS B.V., AMSTERDAM, NL, vol. 817, no. 1-3, 10 August 2007 (2007-08-10), pages 161 - 162, XP022192117, ISSN: 0166-1280, DOI: 10.1016/J.THEOCHEM.2007.04.030**
- **YOUSEF TIZABI ET AL: "Nicotine and the nicotinic cholinergic system in COVID-19", THE FEBS JOURNAL, 25 August 2020 (2020-08-25), GB, XP055731469, ISSN: 1742-464X, DOI: 10.1111/febs.15521**
- **LAGOUMINTZIS GEORGE ET AL: "Nicotinic cholinergic system and COVID-19: In silico identification of interactions between [alpha]7 nicotinic acetylcholine receptor and the cryptic epitopes of SARS-Co-V and SARS-CoV-2 Spike glycoproteins", FOOD AND CHEMICAL TOXICOLOGY, vol. 149, 24 January 2021 (2021-01-24), GB, pages 112009, XP055813944, ISSN: 0278-6915, Retrieved from the Internet <URL:https://reader.elsevier.com/reader/sd/pii/S0278691521000430?token=C613862092EC822EE15142AF583DA6CB3F051D593B935AC2F581E239431A07E740D6AD5CFD1EC4AFCE3A05A5D5E30190&originRegion=eu-west-1&originCreation=20210615100843> DOI: 10.1016/j.fct.2021.112009**


(52) Cooperative Patent Classification (CPC): (Cont.)

C-Sets
**A61K 31/506, A61K 2300/00;**
**A61K 31/55, A61K 2300/00;**
**A61K 33/34, A61K 2300/00**

## Description

### CROSS REFERENCE TO RELATED APPLICATIONS

This application claims the benefit of U.S. Provisional Application No. 63/016,886, filed April 28, 2020.

### TECHNICAL FIELD

The present disclosure relates generally to the use of nicotinic acetylcholine receptor (nAChR) agonists for the prevention, inhibition, or treatment of viral respiratory infections, e.g., coronavirus infections (e.g., coronavirus 229E, NL63, OC43, HKU1, MERS-CoV, SARS-CoV, SARS-CoV-2) via local administration to the upper and/or lower respiratory tract. In particular, the present invention provides a compound for use in a method of inhibiting a coronavirus infection in an individual in need thereof, wherein the compound is a nAChR agonist, or a pharmaceutically acceptable salt thereof, wherein the nAChR agonist is varenicline, wherein the method comprises locally administering the nAChR agonist, or a pharmaceutically acceptable salt thereof, into the respiratory tract of the individual in need thereof, and wherein the method further comprises locally administering a pharmaceutically acceptable salt of copper into the respiratory tract of the individual in need thereof. The present invention also provides a pharmaceutical formulation comprising a nicotinic acetylcholine receptor (nAChR) agonist and a pharmaceutically acceptable salt of copper, wherein the nAChR agonist is varenicline, or a pharmaceutically acceptable salt thereof.

### BACKGROUND

COVID-19 is caused by a novel coronavirus (SARS-CoV-2). On March 11, 2020, the World Health Organization declared the COVID-19 (coronavirus disease 2019) outbreak a pandemic. According to the World Health Organization on March 17, 2020, approximately 170,000 confirmed cases of COVID-19 caused by SARS-CoV-2 had been reported, including an estimated 7,000 deaths in approximately 150 countries. About one year later, as of March 23, 2021, the Center for Systems Science and Engineering (CSSE) at Johns Hopkins University (JHU) reported over 124 million total confirmed cases worldwide, with the US reporting the greatest number of cases - approximately 30 million. Total deaths worldwide were approximately 2.7 million, with over half a million deaths in the US. In general, symptoms of COVID-19 include fever, cough, and shortness of breath.

Potential ways in which the novel coronavirus can be spread include a subject touching the skin of other people or objects that are contaminated with infectious virus-containing droplets and then touching their eye(s), nose, or mouth. The novel coronavirus can also be spread when an infected person coughs, sneezes, or talks, thereby producing droplets of saliva or discharge into the air. These airborne droplets containing viral particles may be inhaled by another person, thereby spreading the virus and continuing the infection cycle. Also, the clinical picture of COVID-19 is not fully known. Reported illnesses have ranged from very mild (including some people who are asymptomatic) to severe, including illness resulting in death. Because the etiology of the infection has not yet been fully determined, no specific prevention and treatment recommendations have yet been validated, although speculation abounds regarding possible preventative and treatment regimens.

CHANGEUX JEAN-PIERRE ET AL: "A nicotinic hypothesis for Covid-19 with preventive and therapeutic implications", QEIOS, 21 April 2020 (2020-04-21) discloses a hypothesis that the nicotinic acetylcholine receptor (nAChR) plays a key role in the pathophysiology of Covid-19 infection and might represent a target for the prevention and control of Covid-19 infection.

KONSTANTINOS FARSALINOS ET AL: "Smoking, vaping and hospitalization for COVID-19", QEIOS, 3 April 2020 (2020-04-03), discloses that there is one study which reported that current smoking is associated with higher odds of severe COVID-19.

YOUSEF TIZABI ET AL: "Nicotine and the nicotinic cholinergic system in COVID-19", THE FEBS JOURNAL, 25 August 2020 (2020-08-25), discloses a potential therapeutic role for nicotine, nicotinic agonists, or positive allosteric modulators of nicotinic cholinergic receptors in COVID-19.

US 2020/281972 A1 discloses formulations containing copper ions and methods of treating underlying infections and conditions caused by coronavirus, particularly COVID-19, and influenzas, particularly influenza A and/or influenza B using such formulations.

### SUMMARY

The invention is defined according to the appended set of claims. The references to methods of treatment in this description are to be interpreted as references to the compounds, pharmaceutical compositions and medicaments of the present invention for use in a method for treatment of the human (or animal) body by therapy."

The present disclosure provides a compound for use in a method of inhibiting a coronavirus infection in an individual in need thereof, wherein the compound is a nAChR agonist, or a pharmaceutically acceptable salt thereof, wherein the nAChR agonist is varenicline, wherein the method comprises administering the nAChR agonist, or a pharmaceutically acceptable salt thereof, into a respiratory tract of the individual in need thereof, and wherein the method further comprises locally administering a pharmaceutically acceptable salt of copper into the respiratory tract of the individual in need thereof.

Additional embodiments, features, and advantages of the invention will be apparent from the following detailed description and through practice of the invention.

## BRIEF DESCRIPTION OF THE DRAWINGS

FIG. 1 shows a diagram of the nose.

FIG. 2 shows a diagram of the respiratory system.

FIG. 3 shows a graph of percent infection of Caco-2 cells with SARS-CoV-2 after 24 hours of exposure to the virus (left y-axis), and percent viability of the Caco-2 cells (right y-axis), as a function of the concentration of varenicline tartrate tested in the assay (x-axis).

FIG. 4 shows a graph of percent infection of Caco-2 cells with SARS-CoV-2 after 24 hours of exposure to the virus (left y-axis), and percent viability of the Caco-2 cells (right y-axis), as a function of the concentration of copper chloride tested in the assay (x-axis).

FIG. 5 shows a graph of percent infection of Caco-2 cells with SARS-CoV-2 after 24 hours of exposure to the virus (left y-axis), and percent viability of the Caco-2 cells (right y-axis), as a function of the concentration of varenicline tartrate tested in the assay (x-axis), wherein 0.3 $\mu$M copper chloride is present in the solution.

## DETAILED DESCRIPTION

The invention is as defined in the appended claims. The methods of the present disclosure involve administering nAChR agonist compositions to the upper and/or lower respiratory tract, such as the nasal cavity, bronchi, oral mucosa, lungs and the like. While not wishing to be bound by theory, the nAChR agonist compositions may be effective in the inhibiting infection by respiratory viruses, such as coronavirus, including SARS-CoV-2 or SARS-CoV-1 by sterically or allosterically competing with the virus at the nAChR. While not wishing to be bound by theory, the nAChR agonist compositions may be effective in the inhibiting infection by respiratory viruses, such as coronavirus, including SARS-CoV-2 or SARS-CoV-1 by binding to the virus spike protein.

The present disclosure therefore provides a compound for use in a method of inhibiting infection by a coronavirus, such as SARS-CoV-2 or SARS-CoV-1, by locally administering an effective amount of a nAChR agonist, or a pharmaceutically acceptable salt thereof, to an individual via the respiratory tract (e.g., a nasal cavity) of the individual. The nAChR agonist is varenicline. The method further comprises locally administering a pharmaceutically acceptable salt of copper into the respiratory tract of the individual in need thereof. The present disclosure also provides a method of inhibiting SARS-CoV-2 infection, comprising administering a nAChR agonist to a cell capable of being infected with SARS-CoV-2, although the appended claims are not directed to this method. Further details of this method are provided below as it is considered useful for understanding the present invention.

The present invention also provides a pharmaceutical formulation comprising a nicotinic acetylcholine receptor (nAChR) agonist and a pharmaceutically acceptable salt of copper, wherein the nAChR agonist is varenicline, or a pharmaceutically acceptable salt thereof.

While treatment regimens to date are focused on systemic administration (e.g., orally ingested dosage forms), local administration of an nAChR agonist to the respiratory tract may offer substantial advantages over systemic administration, in that effective amounts of nAChR agonist can be administered to the site of coronavirus infection (e.g., respiratory tract). Additionally, local administration achieves effective amounts of drug at the site of infection without the side effects associated with systemic administration. In certain cases, it may not be possible to achieve effective concentrations at the site of infection (e.g., the respiratory tract) when drugs are administered systemically. Further if effective amounts can be achieved via systemic administration, the doses required may cause adverse side effects that further worsen an individual's overall health.

The respiratory tract includes the nasal cavity, pharynx, larynx, primary bronchi, and lungs. The major passages and structures of the upper respiratory tract include the nasal cavity, mouth, throat (pharynx), and voice box (larynx). The major passages and structures of the lower respiratory tract include the windpipe (trachea) primary bronchi, and, lungs. And, within the lungs, the bronchi, bronchioles, and alveoli. Local administration to the respiratory tract (including the upper and/or lower respiratory tract), refers to administration of the nAChR agonist (or other co-administered compounds) by

means other than via the circulatory system (e.g., ingestion of a tablet or pill or intravenous delivery or injection). For example, local administration via inhalation (e.g., with a nebulizer, etc.) or e.g., via a spray or jet of liquid.

The cell may be one or more cells capable of being infected with SARS-CoV-2 or SARS-CoV-1. Such a cell or cells may be in an individual, or may be a cell *in vitro,* for example, an isolated cell, or a cell that is part of a cell population or cell culture. An "individual" or "subject" is a mammal, e.g., a human, mouse, rat, guinea pig, dog, cat (e.g., domestic or zoo cats), horse, cow, pig, or non-human primate, such as a monkey, chimpanzee, baboon or rhesus macaque.

Inhibiting infection by a coronavirus, such as SARS-CoV-2 or SARS-CoV-1or inhibiting a coronavirus infection, such as SARS-CoV-2 or SARS-CoV-1 infection, refers to the ability of an nAChR agonist to prevent or reduce invasion by a coronavirus (e.g., SARS-CoV-2, ARS-CoV-1, etc.) into cells to thereby cause COVID-19 or related infection, either in an individual or *in vitro,* or to prevent or reduce a coronavirus, such as SARS-CoV-2 or SARS-CoV-1, from spreading from infected cells to uninfected cells to thereby prolong an existing infection, either within an infected individual or in vitro, or to prevent a coronavirus, such as SARS-CoV-2 or SARS-CoV-1, from replicating within an infected cell. The ability of an nAChR agonist to inhibit infection may also be demonstrated by a lack of symptoms associated with coronavirus, e.g., SARS-CoV-2, in individuals exposed to the virus or a reduction in viral load versus viral loads typical of infected individual. Individuals exposed to the virus may include healthcare workers (e.g., physicians, nurses, or any other individuals who work in a research, hospital or clinical setting), custodial staff (e.g., janitors, laundry services, etc.), caregivers for individuals known to have tested positive for coronavirus infection (e.g., SARS-CoV-2 or SARS-CoV-1) or exhibiting one or more symptoms of coronavirus (e.g., SARS-CoV-2 or SARS-CoV-1), people living in the same household with a person that has become infected, etc. Symptoms of coronavirus include fever, nausea, diarrhea, myalgia, difficulty breathing, acute respiratory distress, anosmia, Guillain-Barré syndrome, confusion, headache, and necrotizing encephalitis. The inhibition of infection and/or the related symptoms, including the amelioration of symptoms, can be based on objective or subjective parameters, which may include the results of a physical examination and/or laboratory testing (e.g., testing for viral load, antibodies to the virus, etc.).

COVID-19 is caused by a new to humans coronavirus, SARS-CoV-2. Coronaviruses are a large family of viruses that are common in people and many different species of animals, including camels, cattle, cats, and bats. Rarely, animal (non-human) coronaviruses can infect people and then spread between people, such as MERS-CoV, SARS-CoV-1, and SARS-CoV-2. The SARS-CoV-2, MERS-CoV and SARS-CoV-1 viruses are betacoronaviruses.

Early on, many of the patients at the epicenter of the COVID-19 outbreak in Wuhan, Hubei Province, China had some link to a large seafood and live animal market, suggesting animal-to-person spread. Later, a growing number of patients reportedly did not have exposure to animal markets, suggesting person-to-person spread. Person-to-person spread was also reported outside Hubei and in countries outside China. Most countries now have ongoing community spread with SARS-CoV-2.

Human coronaviruses usually spread from an infected person to others through the air by coughing and sneezing; close personal contact, like touching or shaking hands; touching an object or surface with the virus on it, then touching your mouth, nose, or eyes before washing your hands.

*Nicotinic Acetylcholine Receptor (nAChR) Agonists*

nAChRs are a class of pentameric ligand-gated ion channels that have high affinity and selectivity for both nicotine and acetylcholine (which resembles nicotine in its protonated form) and comprise combinations of alpha and beta subunits. Examples of nAChR subtypes include, but are not limited to alpha7, alpha3beta4, alpha4beta2, alpha3alpha5-beta4, and alpha4alpha6beta2. An important nAChR receptor subtype involved in instigating the nasolacrimal reflex, for example, is the alpha4beta2 subtype located on the trigeminal nerve endings in the nasal mucosa. There are both neuronal and non-neuronal (including solitary chemosensory cells) nAChR subtypes located within the nasal mucosa and airway. The nAChR agonist of the present invention is varenicline.

Administration of a nAChR agonist locally to the respiratory tract (e.g., directly to the nasal cavity or lungs) will deliver a localized dose to epithelial tissue (e.g., the nasal epithelium, trachea, lung alveoli), allowing a large population of the receptors to be occupied or desensitized and to compete with SARS-CoV-2 or SARS-CoV-1 infection directly at the site of viral entry into the human body. Concentrations achieved by delivering the nicotinic agonist directly to the upper respiratory tract (e.g., nasal mucosa, etc.) or lower respiratory tract cannot be achieved by systemic administration via transdermal, oral, or intravenous delivery based on intolerable or dangerous side effects associated with the systemic dose that would need to be delivered to achieve the same local upper respiratory tract (e.g., nasal mucosa, etc.) or lower respiratory tract concentrations from a local (e.g., nasal) administration of the nAChR agonist.

Administration of a nAChR agonist locally to the respiratory tract (e.g., directly to the nasal cavity or lungs) will deliver a localized dose to epithelial tissue (e.g., the nasal epithelium, trachea, lung alveoli), and may bind to the spike region of the SARS-CoV-2 or SARS-CoV-1 virus. Concentrations achieved by delivering the nicotinic agonist directly to the upper respiratory tract (e.g., nasal mucosa, etc.) or lower respiratory tract cannot be achieved safely by systemic administration via transdermal, oral, or intravenous delivery based on intolerable or dangerous side effects associated

with the systemic dose that would need to be delivered to achieve the same local upper respiratory tract (e.g., nasal mucosa, etc.) or lower respiratory tract concentrations from a local (e.g., nasal) administration of the nAChR agonist.

Local respiratory delivery (e.g., nasal administration) of a nAChR agonist may be alone or in combination with a copper containing solution, for example, an aqueous solution of a copper salt (e.g., copper chloride, copper sulfate, etc.). The method of the present invention further comprises locally administering a pharmaceutically acceptable salt of copper into the respiratory tract of the individual in need thereof.

Local respiratory delivery (e.g., nasal administration) of a nAChR agonist, wherein the nAChR agonist is varenicline, or a pharmaceutically acceptable salt thereof, may be alone or in combination with a systemically administered agonist such as varenicline, encenicline, or simpinicline.

Administration of an nAChR agonist may occupy or desensitize the receptor. Receptor desensitization results in reduced response to agonists even at higher agonist concentrations. While the nAChR receptor is occupied by an agonist, and during the period it is desensitized, the susceptibility of an individual to infection by a coronavirus, for example SARS-CoV-2 or SARS-CoV-1, may be diminished. Thus, agonist occupancy and/or desensitization of nAchR receptors may inhibit the infection of an individual by a coronavirus, such as SARS-CoV-2 or SARS-CoV-1. For instance, short term desensitization of the nAChR receptor to an agonist may occur over a 24 hour period after administration of the agonist. The potential for receptor desensitization may potentially allow for a convenient dosing frequency over a period of time in order to maintain occupancy and/or desensitization of the receptor.

A nAChR agonist may be characterized as a full or partial agonist as determined by its ability to activate a given receptor subtype to produce a response as compared to the response at that receptor for acetylcholine (ACh). In general, a nAChR agonist is a full agonist if it evokes a response upon binding to a given receptor that is equal or greater to that of ACh. A nAChR agonist is a partial agonist if it evokes a lower response upon binding to the receptor as compared to the response generated from ACh.

nAChR agonist response, from which receptor activation can be determined can, for example, be generated using an appropriate cell-based assay. Cells designed to express a particular nAChR receptor subtype and generate an electrical current response when bound to and activated by a nAChR agonist can be used to characterize the agonist profile of a compound and the amount of receptor activation thus determined. An example of a generic protocol is described below.

Cells that express a particular human nAChR subtype are first exposed to ACh. ACh binds and activates the receptor, thereby evoking a current. The concentration of the ACh is chosen to elicit the maximum response of the receptor (*e.g.*, 1280 μM ACh). This current is recorded as the ACh response and serves as the 100% nAChR agonist response and to which responses to other nAChR agonists are compared. After washing, the cells are exposed to a nAChR agonist at various concentrations (*e.g.*, 0.1, 0.3, 1, 3, 10, 30, 100, and 300 μM). The current evoked by exposure to the nAChR agonist is measured and recorded for each nAChR concentration. This nAChR agonist response data is then normalized to unity versus the maximal ACh evoked current and plotted as a function of the logarithm of the nAChR agonist concentration. The nAChR agonist response is then calculated as a percentage of the ACh response.

In some cases, the method to determine the relative agonist activity of nAChR agonist comprises conditions wherein the ACh response is evoked from a 1 or more mM ACh solution.

A nAChR agonist evoking a response equal to or greater than the maximum ACh response determined at the same receptor type is a full agonist. In some cases, a nAChR agonist evoking a response of less than 100% of the ACh response may still be characterized a full agonist, taking into account experimental variability. For example, variability between tests or measurement methods, and statistical error, may account for differences in the response results. In some cases, a nAChR agonist evoking 80% to 120% of the ACh response is considered a full agonist. In some cases, a nAChR agonist evoking 99% of the ACh response or greater is considered a full agonist. In some cases, a nAChR agonist evoking 95% of the ACh response or greater is considered a full agonist. In some cases, a nAChR agonist evoking 90% of the ACh response or greater is considered a full agonist. In some cases, a nAChR agonist evoking 85% of the ACh response or greater is considered a full agonist. In some cases, a nAChR agonist evoking 80% of the ACh response or greater is considered a full agonist.

Taking into account experimental variability, if the nAChR agonist evokes less than 100% of the ACh response, then generally the agonist is considered a partial agonist. In some cases, a nAChR agonist evoking less than 95% of the ACh response is considered a partial agonist. In some cases, a nAChR agonist evoking less than 90% of the ACh response is considered a partial agonist. In some cases, a nAChR agonist evoking less than 85% of the ACh response is considered a partial agonist. In some cases, a nAChR agonist evoking less than 80% of the ACh response is considered a partial agonist.

In some cases, a nAChR agonist evoking 5% to 95% of the ACh response is considered a partial agonist. In some cases, a nAChR agonist evoking 5% to 90% of the ACh response is considered a partial agonist. In some cases, a nAChR agonist evoking 5% to 85% of the ACh response is considered a partial agonist. In some cases, a nAChR agonist evoking 5% to 80% of the ACh response is considered a partial agonist.

In some cases, a nAChR agonist evoking 10% to 95% of the ACh response is considered a partial agonist. In

some cases, a nAChR agonist evoking 10% to 90% of the ACh response is considered a partial agonist. In some cases, a nAChR agonist evoking 10% to 85% of the ACh response is considered a partial agonist. In some cases, a nAChR agonist evoking 10% to 80% of the ACh response is considered a partial agonist.

nAChR agonists that generate a low level of electrical activity at relatively high concentrations of agonist may be described as a weak partial agonist. In some cases, a nAChR agonist evoking 30% or less of the ACh response is considered a weak partial agonist. In some cases, a nAChR agonist evoking 25% or less of the ACh response is considered a weak partial agonist. In some cases, a nAChR agonist evoking 20% or less of the ACh response is considered a weak partial agonist. In some cases, the relatively high concentration of nAChR agonist is at least 100 μM. In some cases, the relatively high concentration of nAChR agonist is at least 200 μM. In some cases, the relatively high concentration of nAChR agonist is at least 300 μM or greater. For instance, a 300 μM concentration of nAChR agonist that evokes 25% of the maximal Ach-evoked current is considered a weak partial agonist.

In some embodiments, the nAChR agonist is a full agonist. In some embodiments, the nAChR agonist is a partial agonist. In some embodiments, the nAChR agonist is a weak partial agonist.

In all embodiments, the nAChR agonist is varenicline, or a pharmaceutically acceptable salt thereof. In some embodiments, the nAChR agonist, or a pharmaceutically acceptable salt thereof, is an agonist of at least one of the nAChR subtypes selected from alpha7, alpha3beta4, alpha3alpha5beta4, alpha4beta2, and alpha4alpha6beta2, wherein the nAChR agonist is varenicline, or a pharmaceutically acceptable salt thereof. In some embodiments, the nAChR agonist, or a pharmaceutically acceptable salt thereof, is an agonist of at least two of the nAChR subtypes selected from alpha7, alpha3beta4, alpha3alpha5beta4, alpha4beta2, and alpha4alpha6beta2, wherein the nAChR agonist is varenicline, or a pharmaceutically acceptable salt thereof. In some embodiments, the nAChR agonist, or a pharmaceutically acceptable salt thereof, is an agonist of at least three of the nAChR subtypes selected from alpha7, alpha3beta4, alpha3alpha5beta4, alpha4beta2, and alpha4alpha6beta2, wherein the nAChR agonist is varenicline, or a pharmaceutically acceptable salt thereof. In some embodiments, the nAChR agonist, or a pharmaceutically acceptable salt thereof, is an agonist of nAChR subtype alpha3beta4, wherein the nAChR agonist is varenicline, or a pharmaceutically acceptable salt thereof. In some embodiments, the nAChR agonist, or a pharmaceutically acceptable salt thereof, is an agonist of nAChR subtype alpha3alpha5beta4, wherein the nAChR agonist is varenicline, or a pharmaceutically acceptable salt thereof. In some embodiments, the nAChR agonist, or a pharmaceutically acceptable salt thereof, is an agonist of nAChR subtype alpha4beta2, wherein the nAChR agonist is varenicline, or a pharmaceutically acceptable salt thereof. In some embodiments, the nAChR agonist, or a pharmaceutically acceptable salt thereof, is an agonist of nAChR subtype alpha4alpha6beta2, wherein the nAChR agonist is varenicline, or a pharmaceutically acceptable salt thereof. In some embodiments, the nAChR agonist, or a pharmaceutically acceptable salt thereof, is an agonist of nAChR subtype alpha7, wherein the nAChR agonist is varenicline, or a pharmaceutically acceptable salt thereof. In some embodiments, the nAChR agonist is a full agonist of the aforementioned subtypes, wherein the nAChR agonist is varenicline, or a pharmaceutically acceptable salt thereof. In some embodiments, the nAChR agonist is a partial agonist of the aforementioned subtypes, wherein the nAChR agonist is varenicline, or a pharmaceutically acceptable salt thereof. In some embodiments, the nAChR agonist is a weak partial agonist of the aforementioned subtypes, wherein the nAChR agonist is varenicline, or a pharmaceutically acceptable salt thereof.

The terms "alpha7" or "α7" nAChR refer to the homomeric alpha7 subtype, wherein the pentameric subunits of nAChR are composed entirely of alpha7 subunits. Thus, a nAChR agonist that binds and activates nAChR alpha7 is an agonist that binds and activates nAChR homomeric alpha7 receptor.

In all embodiments, the nAChR agonist is varenicline, or a pharmaceutically acceptable salt thereof. In some embodiments, the nAChR agonist, or a pharmaceutically acceptable salt thereof, selectively binds to at least one of the nAChR subtypes selected from alpha7, alpha3beta4, alpha3alpha5beta4, alpha4beta2, and alpha4alpha6beta2. As used herein, "selectively binds" or "is selective for" means that a compound has a higher affinity for the nAChR subtype and/or a lower half-maximal effective concentration (EC50) for that nAChr subtype for at least one reference nAChR subtype. Selectivity may be associated with at least a 5-fold affinity difference in EC50 value, at least a 10-fold affinity difference in EC50 value, at least a 20-fold affinity difference in EC50 value, or at least a 50-fold affinity difference in EC50 value. In some embodiments, the nAChR agonist, or a pharmaceutically acceptable salt thereof, selectively binds to nAChR subtype alpha3beta4. In some embodiments, the nAChR agonist, or a pharmaceutically acceptable salt thereof, selectively binds to nAChR subtype alpha3alpha5beta4. In some embodiments, the nAChR agonist, or a pharmaceutically acceptable salt thereof, selectively binds to nAChR subtype alpha4beta2. In some embodiments, the nAChR agonist, or a pharmaceutically acceptable salt thereof, selectively binds to nAChR subtype alpha4alpha6beta2. In some embodiments, the nAChR agonist, or a pharmaceutically acceptable salt thereof, selectively binds to nAChR subtype alpha7.

Exemplary nAChR agonists contemplated in this disclosure include varenicline, or a pharmaceutically acceptable salt thereof. Compound 1, or a pharmaceutically acceptable salt thereof, is a nAChR agonist not covered by the appended claims.

Varenicline is characterized as a full agonist of the nAChR subtype alpha7 and a partial agonist of subtypes alpha3beta4, alpha4beta2, alpha6beta2, alpha3alpha5beta4, and alpha4alpha6beta2. In all embodiments described

herein, the nAChR agonist is varenicline, or a pharmaceutically acceptable salt thereof. Pharmaceutically acceptable salts of varenicline include varenicline tartrate. Additional information for varenicline may be found in, for example, U.S. Patent 6,410,550, U.S. Patent 6,890,927, U.S. Patent 7,265,119, U.S. Patent 9,504,644, U.S. Patent 9,504,645, U.S. Patent 9,532,944, U.S. Patent 9,597,284, U.S. Patent 10,456,396, and PCT publication WO 2020/014232.

Compound 1, as recited herein, refers to the structure:

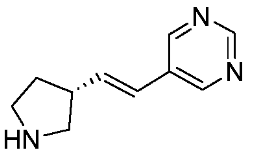
(1)

An alternative structural representation of compound 1 is shown here:

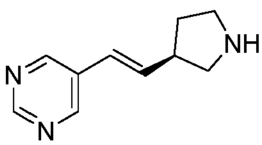

Compound 1 may be also referred to by its chemical name. For instance, compound 1 is also referred to as (R)-5-((E)-2-pyrrolidin-3-ylvinyl)pyrimidine, or variations thereof including 5-{(E)-2-[(3R)-pyrrolidin-3-yl]vinyl}pyrimidine and (R,E)-5-((2-pyrrolidine-3-yl)vinyl)pyrimidine. Compound 1 is also known as simpinicline.

Compound 1 is a full agonist of nAChR subtypes alpha4beta2, alpha7, alpha3beta4, alpha3alpha5beta4, and alpha4alpha6beta2. Compound 1 is a full agonist of nAChR subtypes alpha4beta2, and alpha3beta4.

Compound 1 is a partial agonist of subtype alpha3beta2.

Compound 1 is a weak partial agonist of subtype alpha7. In one example, a 300 μM concentration of compound 1 citrate evoked only 25% of the maximal ACh-evoked current.

Pharmaceutically acceptable salts of compound 1 include galactarate (e.g., hemi-galactarate dihydrate) and citrate (e.g., mono-citrate). Patent related information for compound 1 may be found in U.S. Patent 7,098,331, U.S. Patent 7,714,001, U.S. Patent 8,063,068, U.S. Patent 8,067,443, U.S. Patent 8,604,191, U.S. Patent 9,145,396, U.S. Patent 9,981,949, U.S. Patent 8,633,222, U.S. Patent 10,709,707, PCT publications WO 2017/177024, WO 2020/014217, and WO 2020/014232.

The nAChR agonist is varenicline, or a pharmaceutically acceptable salt thereof. In some embodiments, the nAChR agonist is varenicline tartrate. In some embodiments, the nAChR agonist is varenicline, or a pharmaceutically acceptable salt thereof.

The term nAChR agonist is not intended to include nAChR agonists that cause moderate to severe pain when administered into the nasal cavity of an individual (e.g., nicotine, GTS-21, cytisine, ABT-418). Where GTS-21 and ABT-418 refer to the structures below:

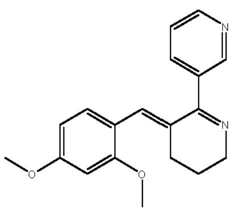
(GTS-21)
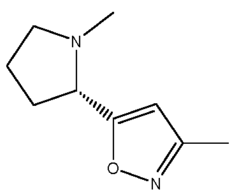
(ABT-418).

*DOSING TIMING AND METHOD OF ADMINISTRATION*

The schedule of doses administered to an individual depends on various considerations including the duration of effectiveness of each dose, the pharmacokinetic profile of the drug, and the effect of the dose on the body. For instance, the period of time between administrations of one or more doses may be extended or decreased, or the period of time between days the subject is administered one or more doses may be extended or decreased. As a non-limiting example, daily administration of one or more doses may include administration 1, 2, 3, 4, 5, or 6 or more times a day. In some embodiments, a dose is administered to the subject in need thereof one to six times daily, two to three times daily, two to four times daily, two to five times daily, two to six times daily, three to four times daily, three to five times daily, three to six times daily, one time daily, two times daily, three times daily, four times daily, five times daily, or six times daily. In some

embodiments, a dose is administered to the subject in need thereof multiple times a day (i.e., more than once per day).

The term "dose", as used herein, may refer to a dose of nAChR agonist. In some of the embodiments described herein, a dose of the nAChR agonist is administered to the subject in need thereof one to six times daily, such as once a day, twice a day, three times a day, four times a day, five times a day, or six times a day, or on an as needed basis (e.g. based on risk of exposure to virus). In some of the embodiments described herein, a dose of the nAChR agonist is administered to the subject in need thereof one to six times daily, two to three times daily, two to four times daily, two to five times daily, two to six times daily, three to four times daily, three to five times daily, three to six times daily, one time daily, two times daily, three times daily, four times daily, five times daily, or six times daily.

In some of the embodiments described herein, the length of time between dosing is increased or decreased. For instance, administration of a dose every 4 hours is modified to administration of a dose every 8 or 12 hours. In other instances, a dose every 4 hours is modified to a dose every 2 or 3 hours. In some embodiments, the dosing frequency may be increased immediately before, during or immediately after potential exposure to coronavirus is highest. For example, in the case of a healthcare worker or other individual working in an essential business may be prescribed more frequent dosing (e.g., fewer hours between doses) immediately before, during, and immediately after a work shift, but decrease dosing (e.g., a greater number of hours between doses) when not working (e.g., while at home and/or sleeping). In some embodiments, the individual is administered a dose every 2-5, 3-5 or 3-4 hours during periods when exposure to coronavirus (e.g., SARS-CoV-2, SARS-CoV-1, etc.) is likely (e.g., during working hours or when caregiving or exposed to an infected person). Periods of likely exposure would include, for example, when caring for an infected individual(s), when in the presence of infected individual(s), during working hours for essential business personnel, or other times when an individual is at heightened risk for coming in contact with an infected individual or site of infection (e.g., locations, surfaces, etc. where coronavirus is likely to be present).

Administration of a single dose may require one administration or multiple administrations of a drug (e.g. multiple sprays of a nAChR agonist). In some of the embodiments described herein, the dose comprises multiple administrations of the nAChR agonist locally to the respiratory tract. In some of the embodiments described herein, the dose comprises multiple administrations of nAChR agonist locally to the upper and lower respiratory tract. In some of the embodiments described herein, the dose comprises multiple administrations of the nAChR agonist locally via a nasal spray (e.g., alone or in combination with another route of administration (e.g., inhaler or nebulizer)). In some of the embodiments described herein, the dose comprises multiple administrations of the nAChR agonist locally via an inhaler or nebulizer. In some embodiments, the nAChR agonist is administered via a nasal nebulizer. In some of the embodiments described herein, the dose comprises multiple administrations of the nAChR agonist to each nostril. In some of the embodiments described herein, the dose comprises multiple administrations of the nAChR agonist to one or both nostrils.

In some of the embodiments described herein, the dose comprises a single administration of the nAChR agonist to each nostril. In some of the embodiments described herein, the dose comprises a single administration of the nAChR agonist, to one nostril.

In some of the embodiments described herein, the nAChR agonist is administered to one nostril per dose. In some of the embodiments described herein, the nAChR agonist is administered to both nostrils per dose.

In some of the embodiments described herein, the nAChR agonist is administered for at least 1 week, at least 2 weeks, at least 3 weeks, at least 4 weeks, at least 1 month, at least 2 months, at least 3 months, at least 4 months, at least 5 months, at least 6 months, at least 7 months, at least 8 months, at least 9 months, at least 10 months, at least 11 months, at least one year, or longer. In some of the embodiments described herein, the nAChR agonist is administered for 2-52 weeks, 2-40 weeks, 2-36 weeks, 2-24 weeks, 2-12 weeks, 2-8 weeks, 4-52 weeks, 4-40 weeks, 4-36 weeks, 4-24 weeks, 4-12 weeks, 4-8 weeks, 5-52 weeks, 5-40 weeks, 5-36 weeks, 5-24 weeks, 5-12 weeks, 5-8 weeks, 6-52 weeks, 6-40 weeks, 6-36 weeks, 6-24 weeks, 6-12 weeks, or 6-8 weeks. In some embodiments, the duration of administration is predicated on the likelihood of the individual's exposure to coronavirus (e.g., SARS-CoV-2 or SARS-CoV-1), such as advised by the World Health Organization (WHO) or national, state, or local advisories. In some embodiments, exposure to the coronavirus may be anticipated such as when an individual enters a medical facility, an area that has a higher concentration of infected individuals, a poorly ventilated enclosure, or is otherwise in close proximity to infected individuals and/or individuals suspected of being infected.

In some of the embodiments described herein, the nAChR agonist is administered for at least 28 days. In some of the embodiments described herein, the nAChR agonist is administered for at least one or more months.

In some of the embodiments described herein, the nAChR agonist is administered for at least 2 months, at least 3 months, at least 4 months, at least 5 months, at least 6 months, at least 7 months, at least 8 months, at least 9 months, at least 10 months, at least 11 months, or at least one year. In some of the embodiments described herein, the nAChR agonist is administered for 2-52 weeks, 2-40 weeks, 2-36 weeks, 2-24 weeks, 2-12 weeks, 2-8 weeks, 4-52 weeks, 4-40 weeks, 4-36 weeks, 4-24 weeks, 4-12 weeks, 4-8 weeks, 5-52 weeks, 5-40 weeks, 5-36 weeks, 5-24 weeks, 5-12 weeks, 5-8 weeks, 6-52 weeks, 6-40 weeks, 6-36 weeks, 6-24 weeks, 6-12 weeks, or 6-8 weeks. In some embodiments, the nAChR agonist is administered seasonally (e.g., for a period of months) or based on risk factors for being exposed to the virus.

In some embodiments, the period of time between the doses is at least 1 hour, at least 2 hours, at least 3 hours, at

least 4 hours, at least 5 hours, at least 6 hours, at least 7 hours, or at least 8 hours. In some embodiments, the period of time between doses is between 1-3 hours, 2-4 hours, 3-6 hours, or 4-8 hours. In some embodiments the period of time between doses is at least 1 hour, at least 2 hours, at least 3 hours, at least 4 hours, at least 5 hours, at least 6 hours, at least 7 hours, or at least 8 hours. In some embodiments the period of time between doses is between 1-3 hours, 2-4 hours, 3-6 hours, or 4-8 hours.

In some embodiments described herein, the time period between administration of doses of a nAChR agonist is less than 5 minutes, between 5-60 minutes, between 30-90 minutes, between 1-3 hours, or between 1-8 hours.

In some of the embodiments described here, the method comprises administering a first dose and one or more subsequent doses of the nAChR agonist. The one or more subsequent doses are administered after a period of time after the first dose. This period of time between the first dose and the next subsequent dose is at least 1 hour, at least 2 hours, at least 3 hours, at least 4 hours, at least 5 hours, at least 6 hours, at least 7 hours, or at least 8 hours. The period of time between the first dose and the next subsequent dose is between 1-3 hours, 2-4 hours, 3-6 hours, or 4-8 hours. The period of time between the one or more subsequent doses is at least 1 hour, at least 2 hours, at least 3 hours, at least 4 hours, at least 5 hours, at least 6 hours, at least 7 hours, or at least 8 hours. The period of time between the one or more subsequent doses is between 1-3 hours, 2-4 hours, 3-6 hours, or 4-8 hours. As noted previously, in some embodiments, the time period between doses may vary, depending on the likely exposure to a coronavirus (e.g., SARS-CoV-2).

*DOSAGE AMOUNT*

In some of the embodiments described herein, the dosages described herein are administered for inhibiting infection by respiratory viruses, such as coronaviruses (e.g., SARS-CoV-2, SARS-CoV-1, etc.). Dosage amounts that are effective may depend on, the individual's health, status, weight, and response to the nAChR agonist, and the judgment of the prescribing health provider. Therapeutically effective amounts are optionally determined by methods including, but not limited to, a dose escalation clinical trial.

The nAChR agonists described herein are administered to an individual susceptible to or otherwise at risk of an infection by a respiratory virus, such as a coronavirus, including SARS-CoV-2 or SARS CoV-1. Amounts that prevent the individual from developing a coronavirus infection or limit the severity of infection (e.g., COVID-19) are considered effective. Such an amount is referred to as an "effective amount or dose."

In some of the embodiments described herein, the amount per dose of the nAChR agonist administered to the individual is 5-4000 μg, 5-1000 μg, 10-2000 μg, 10-700 μg, 100-700 μg, 5-750 μg, 10-750 μg, 100-750 μg, 5-850 μg, 10-850 μg, 100-850 μg, 100-600 μg, 100-500 μg, 200-700 μg, 200-600 μg, 200-500 μg, 300-600 μg, 300-500 μg, 900-4000 μg, 900-3000 μg, 900-2500 μg, 900-2000 μg, 1000-4000 μg, 1000-2500 μg, 1000-2000 μg, 1250-4000 μg, 1250-2500 μg, 1250-2000 μg, 1500-4000 μg, 1500-3000 μg, 1500-2500 μg, 1500-2000 μg, 1800-4000 μg, 1800-3000 μg, 1800-2500 μg, 1800-2250 μg, 2000-4000 μg, 2000-3000 μg, or 2000-2500 μg, or a corresponding amount of a pharmaceutically acceptable salt thereof.

In some of the embodiments described herein, the amount per dose of the nAChR agonist administered to the subject is 900-2500 μg, 1000-2500 μg, 1500-3000 μg, 1800-2500 μg, 1800-2250 μg, or a corresponding amount of a pharmaceutically acceptable salt thereof. In some of the embodiments described herein, the amount per dose of varenicline or compound 1 administered to the subject is 900-2500 μg, 1000-2500 μg, 1500-3000 μg, 1800-2500 μg, 1800-2250 μg, or a corresponding amount of a pharmaceutically acceptable salt thereof.

In some of the embodiments described herein, the amount per dose of varenicline administered to the subject is 5-15 μg, 50-65 μg, 100-125 μg, or a corresponding amount of a pharmaceutically acceptable salt thereof.

In some of the embodiments described herein, the amount per dose of compound 1 administered to the subject is 150-300 μg, 900-1200 μg, 2100-2400 μg, or a corresponding amount of a pharmaceutically acceptable salt thereof.

In some of the embodiments described herein, the pharmaceutically acceptable salt of the nAChR agonist is administered.

The volume of the pharmaceutical formulation administered to a subject depends on various factors, including the route of administration and the type of delivery device. For nasal administration, the volume of the pharmaceutical formulation should be sufficient to deliver the effective amount of the drug to the nasal cavity. Too little a volume might result in the drug not reaching the nasal cavity. On the other hand, the volume should not be so large as to be impractical, uncomfortable, or too difficult to administer to the subject. In addition, too large of a volume may result in the pharmaceutical formulation being delivered to areas of the body not intended for delivery. This can result in waste of the pharmaceutical formulation or irritation of tissues. For instance, reducing a dose volume from 200 μL to 100 μL may reduce the incidence of irritation of the upper throat/soft palate by reducing post-nasal drip after instillation. Likewise, the volume of a pharmaceutical formulation administered via an inhaler or nebulizer should be sufficient for the nAChR agonist to reach the upper respiratory tract, the lower respiratory tract or both the upper and lower respiratory tract.

When volumes of are referred to herein (e.g., microliters (μL), milliliters (mL), etc.) in connection with dosage amounts and concentrations, these volumes are referring to a volume of liquid that is being delivered to an individual. For

example, the administration of a volume of 150 μL via a nasal pump refers to 150 μL of the liquid in the pump reservoir being aerosolized and delivered the nasal cavity of the individual. The volume of a dose is not intended to refer to a volume of gas.

In some of the embodiments described herein, the nAChR agonist, or a pharmaceutically acceptable salt thereof, is administered in a pharmaceutical formulation for nasal administration, and the total volume of the pharmaceutical formulation per dose is 50-250 μL, 75-125 μL, 150-250 μL, or 175-225 μL.

In some of the embodiments described herein, the nAChR agonist, or a pharmaceutically acceptable salt thereof, is administered in a pharmaceutical formulation for nasal administration, and the total volume of the pharmaceutical formulation per dose is about 50 μL, about 75 μL, about 100 μL, about 125 μL, about 150 μL, about 175 μL, about 200 μL, about 225 μL, or about 250 μL. In some of the embodiments described herein, the nAChR agonist, or a pharmaceutically acceptable salt thereof, is administered in a pharmaceutical formulation for nasal administration, and the total volume of the pharmaceutical formulation per dose is about 50 μL, 100 μL, or about 200 μL.

In some of the embodiments described herein, the nAChR agonist, or a pharmaceutically acceptable salt thereof, is administered in a pharmaceutical formulation for nasal administration, and the total volume of the pharmaceutical formulation per nostril is 50-250 μL, 75-125 μL, 150-250 μL, or 175-225 μL.

In some of the embodiments described herein, the nAChR agonist, or a pharmaceutically acceptable salt thereof, is administered in a pharmaceutical formulation for nasal administration, and the total volume of the pharmaceutical formulation per nostril is about 50 μL, about 75 μL, about 100 μL, about 125 μL, about 150 μL, about 175 μL, about 200 μL, about 225 μL, or about 250 μL.

In some of the embodiments described herein, the nAChR agonist, or a pharmaceutically acceptable salt thereof, is administered in a pharmaceutical formulation for nasal administration comprising between 1 mg/mL and 40 mg/mL, between 1 mg/mL and 30 mg/mL, between 1 mg/mL and 20 mg/mL, between 1 mg/mL and 10 mg/mL, between 1 mg/mL and 5 mg/mL, 2 mg/mL and 40 mg/mL, between 2 mg/mL and 30 mg/mL, between 2 mg/mL and 20 mg/mL, between 2 mg/mL and 10 mg/mL, between 2 mg/mL and 5 mg/mL, 5 mg/mL and 40 mg/mL, between 5 mg/mL and 30 mg/mL, between 5 mg/mL and 20 mg/mL, between 5 mg/mL and 10 mg/mL, or between 5 mg/mL and 15 mg/mL, of the nAChR agonist, or a corresponding amount of a pharmaceutically acceptable salt thereof.

In some of the embodiments described herein, the nAChR agonist, or a pharmaceutically acceptable salt thereof, is administered in a pharmaceutical formulation for nasal administration comprising about 1 mg/mL, about 2 mg/mL, about 3 mg/mL, about 4 mg/mL, about 5 mg/mL, about 6 mg/mL, about 7 mg/mL, about 8 mg/mL, about 9 mg/mL, about 10 mg/mL, about 11 mg/mL, about 12 mg/mL, or about 20 mg/mL of the nAChR agonist, or a corresponding amount of a pharmaceutically acceptable salt thereof.

In some of the embodiments described herein, the nAChR agonist, or a pharmaceutically acceptable salt thereof, is administered in a pharmaceutical formulation for nasal administration comprising between 1 μM and 1 mM, or 10 μM and 10 mM, of the nAChR agonist, or a corresponding amount of a pharmaceutically acceptable salt thereof.

In some of the embodiments described herein, the nAChR agonist, or a pharmaceutically acceptable salt thereof is administered in a pharmaceutical formulation for nasal administration, wherein the concentration of the nAChR agonist per dose is 0.01% (w/v) to 0.5% (w/v), 0.01% (w/v) to 1.0% (w/v), 0.01% (w/v) to 1.5% (w/v), 0.01% (w/v) to 2.0% (w/v), 0.01% (w/v) to 2.5% (w/v), 0.01% (w/v) to 3.0% (w/v), 0.5% (w/v) to 1.0% (w/v), 0.5% (w/v) to 1.5% (w/v), 0.5% (w/v) to 2.0% (w/v), 0.5% (w/v) to 2.5% (w/v), 0.5% (w/v) to 3.0% (w/v), 1.0% (w/v) to 1.5% (w/v), 1.0% (w/v) to 2.0% (w/v), 1.0% (w/v) to 2.5% (w/v), 1.0% (w/v) to 3.0% (w/v), 1.5% (w/v) to 2.0% (w/v), 1.5% (w/v) to 2.5% (w/v), 1.5% (w/v) to 3.0% (w/v), 2.0% (w/v) to 2.5% (w/v), or 2.0% (w/v) to 3.0% (w/v), or a corresponding amount of a pharmaceutically acceptable salt thereof.

In some of the embodiments described herein, varenicline, or a pharmaceutically acceptable salt thereof is administered in a pharmaceutical formulation for nasal administration, wherein the concentration of varenicline per dose is about 0.058% (w/v), or about 0.12% (w/v), or a corresponding amount of a pharmaceutically acceptable salt thereof.

In some of the embodiments described herein, varenicline, or a pharmaceutically acceptable salt thereof, is administered in a pharmaceutical formulation for nasal administration, wherein the concentration of varenicline, per dose is less than about 0.06% (w/v), or less than 0.15% (w/v), or a corresponding amount of a pharmaceutically acceptable salt thereof.

In some of the embodiments described herein, varenicline, or a pharmaceutically acceptable salt thereof, is administered in a pharmaceutical formulation for nasal administration, wherein the concentration of varenicline, per dose is about 0.058% (w/v), or about 0.12% (w/v), or a corresponding amount of a pharmaceutically acceptable salt thereof; and wherein the volume per dose is about 50 μL.

In some of the embodiments described herein, varenicline, or a pharmaceutically acceptable salt thereof, is administered in a pharmaceutical formulation for nasal administration, wherein the concentration of varenicline, per dose is less than about 0.06% (w/v), or less than 0.15% (w/v), or a corresponding amount of a pharmaceutically acceptable salt thereof; and wherein the volume per dose is about 50 μL.

In some of the embodiments described herein, varenicline, or a pharmaceutically acceptable salt thereof, is administered in a pharmaceutical formulation for nasal administration, wherein the concentration of varenicline, per dose is about 0.058% (w/v), or about 0.12% (w/v), or a corresponding amount of a pharmaceutically acceptable salt thereof; and

wherein the volume per dose is about 100 μL. In some of the embodiments herein, the 100 μL dose is delivered as two 50 μL sprays. In some of the embodiments herein, the 100 μL dose is delivered as a single 100 μL spray.

In some of the embodiments described herein, varenicline, or a pharmaceutically acceptable salt thereof, is administered in a pharmaceutical formulation for nasal administration, wherein the concentration of varenicline, per dose is less than about 0.06% (w/v), or less than 0.15% (w/v), or a corresponding amount of a pharmaceutically acceptable salt thereof; and wherein the volume per dose is about 100 μL. In some of the embodiments herein, the 100 μL dose is delivered as two 50 μL sprays. In some of the embodiments herein, the 100 μL dose is delivered as a single 100 μL spray.

In some of the embodiments described herein, the nAChR is a pharmaceutically acceptable salt. In some of the embodiments described herein, the nAChR agonist is varenicline tartrate.

The amount of nAChR agonist free base and the corresponding amount of salt administered to a subject in need thereof may be calculated based on the nAChR agonist concentration of the pharmaceutical formulation and volume of the pharmaceutical formulation administered to the subject need thereof. The following examples illustrate the relationship between concentration and volume of the pharmaceutical formulation, and the amounts of nAChR agonist salt and free base administered to the subject.

For example, a 2.0% compound 1 hemi-galactarate dihydrate solution is equivalent to 20 mg of the salt per 1 mL of solution. The 2.0% compound 1 hemi-galactarate dihydrate solution corresponds to a 1.1% compound 1 free base solution. 1.1% of free base solution is equivalent to 11.1 mg/mL of compound 1 free base.

50 μL of 2.0% compound 1 hemi-galactarate dihydrate solution contains about 1000 μg of compound 1 hemi-galactarate dihydrate, which is equivalent to about 554 μg of compound 1 free base. Likewise, 100 μL of 2.0% compound 1 hemi-galactarate dihydrate solution contains about 2000 μg of compound 1 hemi-galactarate dihydrate, which is equivalent to about 1108 μg of compound 1 free base. Amounts of salt and the corresponding amount of free base may be calculated for other concentrations or volumes in a similar fashion.

In another example, a 2.3% compound 1 mono-citrate solution is equivalent to 23.2 mg of the salt per 1 mL of solution. The 2.3% compound 1 mono-citrate solution corresponds to a 1.1% compound 1 free base solution. 1.1% of free base solution is equivalent to 11.1 mg/mL of compound 1 free base.

50 μL of 2.3% compound 1 mono-citrate solution contains about 1161 μg of compound 1 mono-citrate, which is equivalent to about 554 μg of compound 1 free base. Likewise, 100 μL of 2.3% compound 1 mono-citrate solution contains about 2322 μg of compound 1 mono-citrate, which is equivalent to about 1108 μg of compound 1 free base. Amounts of salt and the corresponding amount of free base may be calculated for other concentrations or volumes in a similar fashion.

In another example, a 0.10% varenicline tartrate solution is equivalent to 1.00 mg of the salt per 1 mL of solution. The 0.10% varenicline tartrate solution corresponds to 0.0585% varenicline free base in solution. 0.0585% of free base solution is equivalent to 0.584 mg/mL of varenicline free base.

50 μL of 0.10% varenicline tartrate solution contains about 50 μg of varenicline tartrate, which is equivalent to about 29.2 μg of varenicline free base. Likewise, 100 μL of 0.10% varenicline tartrate solution contains about 100 μg of varenicline tartrate, which is equivalent to about 58.5 μg of varenicline free base. Amounts of salt and the corresponding amount of free base may be calculated for other concentrations or volumes in a similar fashion.

In another example, a 0.20% varenicline tartrate solution is equivalent to 2.00 mg of the salt per 1 mL of solution. The 0.20% varenicline tartrate solution corresponds to 0.117% varenicline free base in solution. 0.117% of free base solution is equivalent to 1.17 mg/mL of varenicline free base.

50 μL of 0.20% varenicline tartrate solution contains about 100 μg of varenicline tartrate, which is equivalent to about 58.5 μg of varenicline free base. Likewise, 100 μL of 0.20% varenicline tartrate solution contains about 200 μg of varenicline tartrate, which is equivalent to about 117 μg of varenicline free base. Amounts of salt and the corresponding amount of free base may be calculated for other concentrations or volumes in a similar fashion.

The corresponding amount of a salt form may be calculated by multiplying the amount of free base by a multiplication factor. The multiplication factor is calculated by dividing the molecular weight of the salt form by the molecular weight of the free base. For instance, the multiplication factor for converting an amount of compound 1 free base to the mono-citrate salt is 2.096. The multiplication factor for converting an amount of compound 1 free base to the hemi-galactarate dihydrate is 1.805. The multiplication factor for converting an amount of varenicline free base to varenicline tartrate is 1.710.

The corresponding amount of the free base may be calculated by multiplying the amount of a salt form by a multiplication factor. The factor is calculated by dividing the molecular weight of the free base by the molecular weight of the salt form. For instance, the multiplication factor for converting an amount of varenicline tartrate to free base is 0.5846. The multiplication factor for converting an amount of compound 1 mono-citrate to free base is 0.477. The multiplication factor for converting an amount of compound 1 hemi-galactarate dihydrate to free base is 0.554.

In some of the embodiments disclosed herein, the nAChR agonist, or a pharmaceutically acceptable salt thereof, is administered to the nasal cavity of a subject. The pharmaceutical formulations described herein include, but are not limited to, liquids, suspensions, aerosols, gels, ointments, dry powders, creams, pastes, lotions, balms, or nasal sprays.

In some of the embodiments disclosed herein, the nAChR agonist, or a pharmaceutically acceptable salt thereof,

administered into the nasal cavity by a spray pump, syringe, dropper, bottle nebulizer, atomization pump, inhaler, powder spray device, vaporizer, patch, medicated stick, pipette, jet of liquid, or nasal spray bottle.

In some of the embodiments disclosed herein, the pharmaceutical formulation is preservative-free.

*SIDE EFFECTS*

The disclosure provides methods of local administration (intranasal) of the nAChR agonist, or pharmaceutically acceptable salts thereof. Local administration has the advantage over systemic administration (e.g., orally (e.g., tablet, pill, capsule), intravenous, or in a patch) including reducing potential side effects by bypassing the digestive system, limiting the plasma concentration and/or amount of drug that may cross the blood-brain barrier, while, at the same time, achieving a high local concentration of nAChR agonist at receptors in the upper respiratory track, for example, within the nasal cavity (cells of the olfactory epithelium/mucosa and/or the underlying olfactory neurons). In some of the embodiments disclosed herein, the nAChR agonist, or pharmaceutically acceptable salt thereof, administered is not systemically bioavailable. In some of the embodiments disclosed herein, the method does not result in undesired systemic side effects. In some of the embodiments disclosed herein, the method does not result in undesired psychoactive side effects.

*Cmax BLOOD PLASMA CONCENTRATION*

The methods and uses described herein include local administration into the respiratory tract (e.g., nasal cavity (e.g., intranasal administration) or lungs) of the nAChR. Because the disclosed methods and uses are to local intranasal administration, the concentration of the nAChR administered to inhibit viral infection (*e.g.*, varenicline, or pharmaceutically acceptable salt thereof), in the circulating blood plasma is low, compared to concentrations achieved from systemic forms of administration (*e.g.* ingestion of oral formulations or skin patches). Low blood plasma concentrations of nAChR agonists avoids potential undesirable side effects associated with nAChR agonists in systemic circulation, such as nausea, sleep disturbance, constipation, flatulence, vomiting, dermal conditions like rash and pruritus, headaches, abdominal pain, dyspepsia, gastroesophageal reflux disease and dry mouth.

One way to characterize the blood plasma concentration of the nAChR agonist, or pharmaceutically acceptable salt thereof, in a subject is to measure the Cmax - the maximum or peak serum concentration that the nAChR agonist, or pharmaceutically acceptable salt thereof, achieves after the nAChR agonist, or pharmaceutically acceptable salt thereof, is administered to the subject and before the administration of a second dose. Methods of determining Cmax are known in the art. A non-limiting example of a protocol used to determine the pharmacokinetic profile of compound 1, including determining Cmax is provided in Example 2.

In some embodiments described herein, the Cmax is calculated for a subject. In some embodiments, Cmax is calculated from the average Cmax of two or more subjects. In some embodiments, Cmax is calculated from the average Cmax of a subset population.

In some embodiments described herein, the subject in need thereof has a blood plasma Cmax of the nAChR agonist, or a pharmaceutically acceptable salt thereof, of less than 5 ng/mL, of less than 4 ng/mL, of less than 3 ng/mL, or of less than 2 ng/mL.

In some embodiments described herein, the subject in need thereof has a blood plasma Cmax of the nAChR agonist, or a pharmaceutically acceptable salt thereof, of less than 5 ng/mL, of less than 4 ng/mL, of less than 3 ng/mL, or of less than 2 ng/mL; and wherein the subject in need thereof was administered a dose comprising between 10 μg to 150 μg, 10 μg to 100 μg, 10 μg to 50 μg, 50 μg to 150 μg, 50 μg to 100 μg, 100 μg to 1500 μg, 100 μg to 600 μg, 200 μg to 400 μg, 400 μg to 600 μg, or 750 μg to 1200 μg of the nAChR agonist, or a corresponding amount of the pharmaceutically acceptable salt thereof.

In some embodiments described herein, the subject in need thereof has a blood plasma Cmax of compound 1, or a pharmaceutically acceptable salt thereof, of less than 5 ng/mL, of less than 4 ng/mL, of less than 3 ng/mL, or of less than 2 ng/mL; and wherein the subject in need thereof was administered a dose comprising between 100 μg to 1500 μg, 100 μg to 600 μg, 200 μg to 400 μg, 400 μg to 600 μg, or 750 μg to 1200 μg of compound 1, or a corresponding amount of the pharmaceutically acceptable salt thereof.

In some embodiments described herein, the subject in need thereof has a blood plasma Cmax of compound 1, or a pharmaceutically acceptable salt thereof, of less than 5 ng/mL, of less than 4 ng/mL, of less than 3 ng/mL, or of less than 2 ng/mL; and wherein the subject in need thereof was administered a dose comprising between 150 μg to 300 μg, 900 μg to 1200 μg, 2100 μg to 2400 μg of compound 1, or a corresponding amount of the pharmaceutically acceptable salt thereof.

In some embodiments described herein, the subject in need thereof has a blood plasma Cmax of varenicline, or a pharmaceutically acceptable salt thereof, of less than 5 ng/mL, of less than 4 ng/mL, of less than 3 ng/mL, or of less than 2 ng/mL; and wherein the subject in need thereof was administered a dose comprising between 5 μg to 15 μg, 50 μg to 65 μg, 100 μg to 125 μg of varenicline, or a corresponding amount of the pharmaceutically acceptable salt thereof.

Throughout the present disclosure, amounts of nAChR agonists disclosed refer to the amount of nAChR agonist

free form free form present in the formulation. The term "corresponding amount" as used herein refers to the amount of a pharmaceutically acceptable salt of a nAChR agonist required to obtain the amount of nAChR free form free form recited in the formulation. It would be clear to one of skill in the art how to calculate the "corresponding amount" of the salt of a compound, such as the corresponding amount of the pharmaceutically acceptable salt of compound 1, taking into account the difference in molecular weight between the free form of a compound and a salt form. For example, 175.24 g of compound 1 free base, would correspond to 316.34 g of the hemi-galactarate dihydrate salt or 367.36 of the mono-citrate salt. In another example, 211.267 g of varenicline free base, would correspond to 361.354 of the varenicline tartrate salt.

*COMBINATION WITH COPPER SALTS*

The methods disclosed herein further comprises locally administering a pharmaceutically acceptable salt of copper into the respiratory tract of the individual in need thereof, such as copper chloride or copper sulfate. The pharmaceutically acceptable salt of copper, may be administered separately from the nAChR agonist or may be administered simultaneously in a single dosage form.

The disclosure also provides a pharmaceutical formulation comprising a nicotinic acetylcholine receptor (nAChR) agonist and a pharmaceutically acceptable salt of copper, wherein the nAChR agonist is varenicline, or a pharmaceutically acceptable salt thereof. In some of the embodiments described herein, the pharmaceutical formulation is preservative-free. The pharmaceutical formulation may be formulated for local nasal administration via a nasal spray bottle or nebulizer.

In some of the embodiments described herein, the pharmaceutically acceptable salt of copper, is administered in a pharmaceutical formulation for nasal administration comprising a concentration of about 1 μM, about 2 μM, about 3 μM, about 4 μM, about 5 μM, about 6 μM, about 7 μM, about 8 μM, about 9 μM, or about 10 μM of the copper, or a corresponding amount of a pharmaceutically acceptable salt thereof.

In some of the embodiments described herein, the pharmaceutically acceptable salt of copper, is administered in a pharmaceutical formulation for nasal administration comprising a concentration of between 0.001 μM and 500 μM, between 0.001 μM and 100 μM, between 0.001 μM and 50 μM, between 0.001 μM and 10 μM, between 0.01 μM and 500 μM, between 0.01 μM and 100 μM, between 0.01 μM and 50 μM, between 0.01 μM and 10 μM, between 0.1 μM and 500 μM, between 0.1 μM and 100 μM, between 0.1 μM and 50 μM, between 0.1 μM and 10 μM, between 1 μM and 500 μM, between 1 μM and 100 μM, between 1 μM and 50 μM, between 1 μM and 10 μM, between 10 μM and 500 μM, between 10 μM and 100 μM, between 10 μM and 50 μM, or between 100 μM and 500 μM, of the copper, or a corresponding amount of a pharmaceutically acceptable salt thereof. In some of the embodiments described herein, the pharmaceutically acceptable salt of copper, is administered in a pharmaceutical formulation for nasal administration comprising a concentration of between 0.001 μM and 10 μM, or between 1 μM and 10 μM, or a corresponding amount of a pharmaceutically acceptable salt thereof.

In some of the embodiments described herein, the amount per dose of the copper administered to the individual is 0.1 μg - 100 mg, 0.1 μg - 50 mg, 0.1 μg - 10 mg, 0.1 μg - 1 mg, 0.1 μg - 500 μg, 0.1 μg - 250 μg, 0.1 μg - 100 μg, 0.1 μg - 50 μg, 0.1 μg - 10 μg, or a corresponding amount of a pharmaceutically acceptable salt thereof.

In some of the embodiments described herein, the amount per dose of the copper administered to the individual is 1 μg - 100 mg, 1 μg - 50 mg, 1 μg - 10 mg, 1 μg - 1 mg, 1 μg - 500 μg, 1 μg - 250 μg, 1 μg - 100 μg, 1 μg - 50 μg, 1 μg - 10 μg, or a corresponding amount of a pharmaceutically acceptable salt thereof.

In some of the embodiments described herein, the amount per dose of the copper administered to the individual is 10 μg - 100 mg, 10 μg - 50 mg, 10 μg - 10 mg, 10 μg - 1 mg, 10 μg - 500 μg, 10 μg - 250 μg, 10 μg - 100 μg, 10 μg - 50 μg, or a corresponding amount of a pharmaceutically acceptable salt thereof.

In some of the embodiments described herein, the amount per dose of the copper administered to the individual is 100 μg - 100 mg, 100 μg - 50 mg, 100 μg - 10 mg, 100 μg - 1 mg, 100 μg - 500 μg, 100 μg - 250 μg, or a corresponding amount of a pharmaceutically acceptable salt thereof.

In some of the embodiments described herein, the amount per dose of the copper administered to the individual is 1 mg - 100 mg, 1 mg - 50 mg, 1 mg - 10 mg, 1 mg - 5 mg, or a corresponding amount of a pharmaceutically acceptable salt thereof.

In some of the embodiments described herein, the amount per dose of the copper administered to the individual is 60 ng - 30 mg, 60 ng - 6 mg, 60 ng - 3 mg, 60 ng - 0.60 mg, 0.60 μg - 30 mg, 0.60 μg - 6 mg, 0.60 μg - 3 mg, 0.60 μg - 0.60 mg, 6 ng - 30 mg, 6 μg - 6 mg, 6 ng - 3 mg, 6 μg - 0.60 mg, 64 μg - 30 mg, 64 μg - 6 mg, 64 μg - 3 mg, 64 μg - 0.60 mg, 0.6 mg - 30 mg, 0.6 mg - 6 mg, 0.6 mg - 3 mg, 6 mg - 30 mg, or a corresponding amount of a pharmaceutically acceptable salt thereof.

The pharmaceutical formulation comprises a nicotinic acetylcholine receptor (nAChR) agonist, or a pharmaceutically acceptable salt thereof, and a pharmaceutically acceptable salt of copper, wherein the nAChR agonist is varenicline.

In some embodiments, the pharmaceutical formulation comprises a nicotinic acetylcholine receptor (nAChR) agonist, or a pharmaceutically acceptable salt thereof, and copper sulfate or copper chloride, wherein the nAChR agonist

is varenicline.

In some embodiments, the pharmaceutical formulation comprises a nicotinic acetylcholine receptor (nAChR) agonist, or a pharmaceutically acceptable salt thereof, and copper sulfate or copper chloride, wherein the nAChR agonist is varenicline; and wherein the pharmaceutical formulation comprises between 100 μg and 3 mg of copper, or a corresponding amount of the pharmaceutically acceptable salt thereof.

In some embodiments, the pharmaceutical formulation comprises a nicotinic acetylcholine receptor (nAChR) agonist, or a pharmaceutically acceptable salt thereof, and copper sulfate or copper chloride, wherein the nAChR agonist is varenicline; and wherein the pharmaceutical formulation comprises between 100 μg and 3 mg of copper, and between 5 and 4000 μg of the nAChR agonist, or a corresponding amount of a pharmaceutically acceptable salt thereof, per dose.

In some embodiments, the pharmaceutical formulation comprises a nicotinic acetylcholine receptor (nAChR) agonist, or a pharmaceutically acceptable salt thereof, and copper sulfate or copper chloride, wherein the nAChR agonist is varenicline; and wherein the pharmaceutical formulation comprises between 100 μg and 3 mg of copper, and between 5 and 4000 μg of the nAChR agonist, or a corresponding amount of a pharmaceutically acceptable salt thereof, per dose, and wherein the pharmaceutical formulation is preservative-free.

In some embodiments, the pharmaceutical formulation comprises a nicotinic acetylcholine receptor (nAChR) agonist, or a pharmaceutically acceptable salt thereof, and copper sulfate or copper chloride, wherein the nAChR agonist is varenicline; and wherein the pharmaceutical formulation comprises copper at a concentration of between 1 μM and 10 μM, or a corresponding amount of the pharmaceutically acceptable salt thereof.

In some embodiments, the pharmaceutical formulation comprises a nicotinic acetylcholine receptor (nAChR) agonist, or a pharmaceutically acceptable salt thereof, and copper sulfate or copper chloride, wherein the nAChR agonist is varenicline; and wherein the pharmaceutical formulation comprises copper at a concentration of between 1 μM and 10 μM, or a corresponding amount of the pharmaceutically acceptable salt thereof, and between 5 and 4000 μg of the nAChR agonist, or a corresponding amount of a pharmaceutically acceptable salt thereof, per dose.

In some embodiments, the pharmaceutical formulation comprises a nicotinic acetylcholine receptor (nAChR) agonist, or a pharmaceutically acceptable salt thereof, and copper sulfate or copper chloride, wherein the nAChR agonist is varenicline; and wherein the pharmaceutical formulation comprises copper at a concentration of between 1 μM and 10 μM, or a corresponding amount of a pharmaceutically acceptable salt thereof, and between 5 and 4000 μg of the nAChR agonist, or a corresponding amount of a pharmaceutically acceptable salt thereof, per dose, and wherein the pharmaceutical formulation is preservative-free.

The nAChR agonist is varenicline or a pharmaceutically acceptable salt thereof. In some embodiments, the varenicline salt is varenicline tartrate. In certain of the pharmaceutical formulation embodiments described herein, the nAChR agonist is varenicline.

As used herein, the term "about" is used synonymously with the term "approximately." Illustratively, the use of the term "about" with regard to an amount indicates values slightly outside the cited values, *e.g.*, plus or minus 0.1% to 20%, plus or minus 0.1% to 10%, plus or minus 0.1% to 5%, or plus or minus 0.1% to 2%.

A statistically significant change, as used herein, to describe a subject's response to treatment may be calculated from an increase or decrease in the subject's score in a given assay. Non-limiting examples of determining whether the change in a subject's score is statistically significant and whether two sets of data are significantly different from each other include calculations based on an ANCOVA model, and statistical hypothesis tests such as t-tests and non-parametric Wilcoxon rank sum tests. Other models and statistical hypothesis tests well-known in the art are contemplated.

## EXAMPLES

The following specific examples are to be construed as merely illustrative, and not limitative of the remainder of the disclosure in any way whatsoever. Examples 1-6 are provided as they are considered useful for understanding the invention.

Example 1 (provided for information only): In Vitro Activity of nAChR Agonists On Various nAChR Subtypes

This example describes experiments to characterize the effects of varenicline tartrate and compound 1 hemi-galactarate dihydrate at the human neuronal nicotinic acetylcholine receptors (nAChRs) alpha3beta4, alpha3alpha5-beta4, alpha4beta2 and alpha7 expressed in Xenopus oocytes.

Determination of the agonistic effects of compound 1 hemi-galactarate dihydrate at the alpha3beta4, alpha3alpha5beta4, alpha4beta2 and alpha7 receptors revealed that this compound acts, in the 10 to 100 μM range, as an agonist at these receptors. Noticeable differences were, however, observed in function of the receptor subtype. Namely, compound 1 hemi-galactarate dihydrate was a poor agonist at the human alpha7 receptors evoking at 300 μM only 24 percent of the maximal ACh-evoked current. In contrast, data from alpha4beta2 revealed that compound 1 hemi-galactarate dihydrate evoked currents larger than the maximal ACh responses by about 2 fold. Moreover, exposure to

compound 1 hemi-galactarate dihydrate enhanced the subsequent ACh-evoked currents. Data recorded at alpha3beta4 and at alpha3alpha5beta4 revealed that compound 1 hemi-galactarate dihydrate acts as an agonist at these receptors evoking about 94 percent of the maximal ACh-evoked currents.

Determination of the agonistic properties of varenicline tartrate at the human alpha3beta4, alpha3alpha5beta4, alpha4beta2 and alpha7 nAChRs, confirmed that this compound acts as a partial agonist at the alpha3beta4, alpha3alpha5beta4 and alpha4beta2 with EC50's in the low micromolar range. Results obtained at the human alpha7 receptors confirmed that varenicline tartrate acts as a full agonist at this receptor subtype with an EC50 at about 11 $\mu$M.

Desensitization experiments conducted alpha3beta4, alpha3alpha5beta4, alpha4beta2 and alpha7 nAChRs revealed marked differences in function of the receptor subtypes. Whereas alpha3beta4 and alpha7 receptors were insensitive to sustained exposure of compound 1 hemi-galactarate dihydrate for concentrations up to 300 nM, a marked inhibition was observed for at alpha3alpha5beta4 and alpha4beta2 receptors. Inhibition reached, however, at the highest concentration tested a plateau level suggesting that this compound might cause only partial desensitization within the experimental conditions. Desensitization experiments conducted at alpha3beta4, alpha3alpha5beta4, alpha4beta2 and alpha7 nAChRs revealed the marked differences that can be observed between these different receptor subtypes. Namely, while varenicline tartrate inhibited in the nanomolar (nM) range up to 78% of the alpha4beta2 receptors, exposure to the same concentrations and duration had only a very modest effect at the alpha3beta4 receptors. Co-expression of the alpha5 receptors in the alpha3alpha5beta4 combination increased, however, the sensitivity to varenicline tartrate, causing up to 45% inhibition. The observed plateau effect of varenicline tartrate at this receptor subtype is in good agreement with its partial agonist activity. Importantly, exposure to varenicline tartrate of the alpha7 nAChRs also caused inhibition for concentrations in the hundreds of nanomolar range. Desensitization caused by 300 $\mu$M varenicline tartrate applied during three minutes at the alpha3beta4 or alpha4beta2 nAChRs, confirmed the differences in sensitivity between these two subtypes. Namely, while varenicline tartrate evoked only about 10-20% of the ACh response at the alpha4beta2, a larger current amplitude was observed at the alpha3beta4 receptors. The absence of recovery observed for period as long as 36 minutes confirmed the long-lasting desensitization caused by exposure to varenicline tartrate.

OBSERVATIONS AND MEASUREMENTS

Oocytes preparation

All experiments were carried out at human nAChRs expressed in *Xenopus* oocytes using the method of cDNA expression. *Xenopus* oocytes were prepared and injected using standard procedures.

Briefly, ovaries were harvested from *Xenopus Laevis* females that have been deeply anesthetized by cooling at 4 °C and Tricaine methanesulfonate (MS-222 at a concentration of 150 mg/L) in sodium bicarbonate (300 mg/L). Once anesthetized the animal was decapitated and pithed following the rules of animal rights from the Geneva canton. A small piece of ovary was isolated for immediate preparation while the remaining part was placed at 4 °C in a sterile Barth solution containing in mM NaCl 88, KCl 1, $NaHCO_3$ 2.4, HEPES 10, $MgSO_4.7H_2O$ 0.82, $Ca(NO_3)_2.4H_2O$ 0.33, $CaCl_2.6H_2O$ 0.41, at pH 7.4, and supplemented with 20 $\mu$g/ml of kanamycin, 100 unit/ml penicillin. All recordings were performed at 18 °C and cells superfused with OR2 medium containing in mM: NaCl 88, KCl 2.5, HEPES 5, $CaCl_2.2H_2O$ 1.8, $MgCl_2.6H_2O$ 1, pH 7.4.

Electrophysiological recordings

Currents evoked by ACh or other agonists were recorded using an automated process equipped with standard two-electrode voltage-clamp configuration (TEVC). Data were captured and analyzed using a HiQScreen proprietary data acquisition and analysis software running under Matlab (Mathworks Inc.).

Agonist Preparation

ACh was prepared as a concentrated stock solution ($10^{-1}$ M) in water and then diluted in the recording medium to obtain the desired test concentration. Compounds were dissolved in OR2 as stock solution (1 mM) and the final dilution to the desired concentration was made on the day of the experiment. OR2 medium contains in mM: NaCl 88.5, KCl 2.5, HEPES 5, $CaCl_2.2H_2O$ 1.8, $MgCl_2.6H_2O$ 1, pH 7.4.

The test concentrations of compound 1 hemi-galactarate and varenicline tartrate were (in $\mu$M) 0.1, 0.3, 1, 3, 10, 30, 100, and 300. The test concentration of acetylcholine chloride was 1280 $\mu$M.

Data Analysis and Statistics

For statistical analysis values were computed either with Excel (Microsoft) or Matlab (mathworks Inc.). To obtain mean measurements with standard deviations, all experiments were carried out using at least three cells.

Experimental Procedures

Injections of cDNAs encoding for the human alpha3beta4, alpha3alpha5beta4, alpha4beta2, and alpha7 were performed in at least 95 oocytes were performed using a proprietary automated injection and receptor expression examined at least two days later. Oocytes were poked with two electrodes and their membrane potential maintained, unless indicated, at -80 mV throughout the experiment.

A protocol to determine agonistic activity in the oocytes expressing the nAChRs was developed. The cells were exposed to a series of increasing concentrations of a compound agonist for 10 seconds each at two minute intervals. The current evoked from exposure to the agonist at each concentration was recorded. For oocytes expressing alpha7 nAChRs, exposure to the agonist was reduced to 5 seconds. This process was repeated for each agonist tested against each type of nAChR expressing cell.

AGONISTIC PROPERTIES OF COMPOUND 1 HEMI-GALACTARATE DIHYDRATE

To evaluate the agonistic properties of compound 1 hemi-galactarate dihydrate a protocol of brief exposures to a series of growing concentrations of compound 1 hemi-galactarate dihydrate was used.

Experiments conducted at the human nAChRs confirmed that compound 1 hemi-galactarate dihydrate behaves as an agonist at the considered receptors. Notably, however, compound 1 hemi-galactarate dihydrate evoked larger currents than the endogenous ligand acetylcholine (ACh) at the heteromeric alpha4beta2 receptors and displayed also at higher potency for this receptor subtype.

Compound 1 hemi-galactarate dihydrate acts almost as a full agonist at nAChR alpha3beta4, yielding an $EC_{50}$ of $42.09 \pm 5.36$ μM.

Compound 1 hemi-galactarate dihydrate acts almost as a full agonist at nAChR alpha3alpha5beta4, yielding an EC50 of $32.15 \pm 2.16$ μM.

Compound 1 hemi-galactarate dihydrate acts as a more potent agonist than ACh at nAChR alpha4beta2, yielding an EC50 of $48.82 \pm 17.41$ μM.

Compound 1 hemi-galactarate dihydrate is a more potent agonist than ACh at nAChR alpha7. The $EC_{50}$ of 1261 $\pm$ 500 μM is only an estimation given the small fraction of current evoked by compound 1 hemi-galactarate dihydrate.

AGONISTIC PROPERTIES OF VARENICLINE TARTRATE

Varenicline tartrate acts as a very poor agonist at the human alpha4beta2 receptors and causes a major inhibition of the subsequent ACh-evoked current. In contrast, varenicline tartrate is a full agonist at the alpha7 receptors with an EC50 at about 15 μM. and causes no significant inhibition of the subsequent ACh-evoked current.

Data obtained for varenicline tartrate confirmed that this molecule acts as a partial agonist at the heteromeric alpha3beta4, alpha3alpha5beta4 and alpha4beta2 but was able to evoke responses equivalent to ACh at the alpha7 receptors.

ANTAGONIST PROPERTIES OF COMPOUND 1 HEMI-GALACTARATE DIHYDRATE AND VARENICLINE TARTRATE

Determination of the antagonistic activity of compound 1 hemi-galactarate dihydrate and varenicline tartrate was done using a cumulative exposure in the low concentration range. Results obtained for compound 1 hemi-galactarate dihydrate revealed that this compound inhibits in a concentration-dependent manner the alpha3alpha5beta4 and alpha4beta2 receptor and that inhibition was incomplete and reached a plateau for the highest concentrations tested. In contrast, no significant inhibition was observed either at alpha3beta4 or alpha7 receptors within the range tested in these experiments.

Antagonistic activity of varenicline tartrate was observed at alpha3alpha5beta4, alpha4beta2 and alpha7 receptors whereas the alpha3beta4 receptors displayed hardly any inhibition for concentrations up to 300 nM. Results obtained at the human alpha3beta4 illustrate that varenicline tartrate causes no significant inhibition of the ACh-evoked current up to concentration of about 300 nM. These data are in good agreement with previous report and that varenicline tartrate will act as an agonist only for concentrations in the micromolar range. Experiments conducted at oocytes injected with alpha3alpha5beta4 reveal, however, a different pattern as a small but significant inhibition of the ACh-evoked responses can be observed even for exposures in the nanomolar range. The maximal inhibition reached about 45% at 300 nM. Data obtained for varenicline tartrate at the human alpha4beta2 illustrate perfectly the inhibition caused by a sustained exposure to this molecule. Whereas a progressive inhibition of the ACh-evoked current is clearly observed as a function of the concentration, data reveals that the inhibition reaches a maximal at 78 % which is in good agreement with the partial agonist activity of varenicline tartrate. These results illustrate that varenicline tartrate also causes a desensitization of the

alpha7 nAChRs.

SUSTAINED EXPOSURE TO 300 μM VARENICLINE TARTRATE AND ITS RECOVERY

As it is expected that receptors can be exposed for a sustained duration to a rather high concentration of varenicline tartrate additional experiments were conducted at the human alpha3beta4 and alpha4beta2. These data illustrate the differences in sensitivity of these two nAChR subtypes and that while exposure to 300 μM varenicline tartrate evokes a rather large response at the human alpha3beta4 it causes only a small activation at the alpha4beta2. Moreover, the desensitization caused by varenicline tartrate shows a long-lasting effect that cannot be recovered even after 36 minutes wash.

Altogether, these data illustrate that expression of human nAChRs in *Xenopus* oocytes provides an efficient model for testing the functional agonistic and antagonistic activity for a given compound and that agonist cause desensitization of the receptors at lower concentrations than those required for activation.

Example 2 (provided for information only): Measurement of Maximum Plasma Concentration

This example describes a method of measuring the maximal plasma concentration (Cmax) of a nAChR agonist, or a pharmaceutically acceptable salt thereof, in an individual after administration of a pharmaceutical formulation comprising the nAChR agonist, or a pharmaceutically acceptable salt thereof.

Dosing

A dose of a nAChR agonist, or a pharmaceutically acceptable salt thereof, is administered to an individual. Examples of dose and volume ranges are described herein.

Blood Collection and Sample Processing

A blood sample is collected from an individual before administration of a nAChR agonist, or a pharmaceutically acceptable salt thereof. After administration of the nAChR agonist, or a pharmaceutically acceptable salt thereof, to the individual, multiple blood samples are collected over a period of time at certain intervals. For instance, blood samples are collected at 10 minutes, 20 minutes, 30 minutes, 1 hour, 2 hours, 3 hours, and 4 hours after administration of the nAChR agonist, or a pharmaceutically acceptable salt thereof.

Blood samples (4 mL in K2EDTA tubes) are collected from an arm vein into Vacutainer tubes containing K2EDTA. Blood samples are mixed gently and maintained chilled until centrifuged within 2 hours of collection.

Plasma samples are separated into two equivalent aliquots and labeled with the individual's identification and nominal time point and stored frozen (at < -70°C) until analysis.

Bioanalytical Methods for Pharmacokinetic Samples

Blood samples from the individual are processed to obtain plasma, and plasma concentrations of nAChR agonist, or a pharmaceutically acceptable salt thereof, using liquid chromatography followed by tandem mass spectrometry (LC-MS/MS) bioanalytical method (high performance liquid chromatography [HPLC] with mass spectrometric detection).

Pharmacokinetic Analysis

All pharmacokinetic (PK) analyses and reporting are performed according to generally accepted standard operating procedures and protocol specifications. Pharmacokinetic parameters are calculated by use of generally accepted software tools, for instance Phoenix® WinNonlin® 8.0.

A plasma concentration time plot is created by plotting the concentration of the nAChR agonist, or pharmaceutically acceptable salt thereof, as a function of time. Cmax is calculated by inspection of an individual's plasma concentration time plot. Another Cmax related parameter is the maximum observed concentration of the nAChR agonist, or a pharmaceutically acceptable salt thereof, divided by mg dose, (Cmax/D). The Cmax is calculated for an individual after a treatment. If the Cmax is calculated for more than one individual, then, the arithmetic mean Cmax and median Cmax may also be calculated for the group.

Example 3 (provided for information only): Measured Cmax After Treatment with Compound 1 Hemi-Galactarate Dihydrate

The blood plasma of 30 individuals treated with 1.1% compound 1 free base (2.0% compound 1 hemi-galactarate dihydrate) via a nasal spray (200 μL total volume; about 2216 μg of compound 1 free base) was analyzed using the protocol described in Example 2. The mean Cmax of compound 1 measured was 2.92 ng/mL.

Example 4 (provided for information only): Compound 1 Citrate and Varenicline Tartrate Agonist Activity in Human nAChR Expressed in Xenopus Oocytes

This example describes experiments to characterize the agonist activity of compound 1 citrate at the human neuronal nicotinic acetylcholine receptors (nAChRs) alpha3beta4, alpha3alpha5beta4, alpha4beta2, alpha4alpha6beta2, and alpha7 expressed in Xenopus oocytes. In addition, the effect of varenicline tartrate on alpha4alpha6beta2 expressed in Xenopus oocytes was measured.

Methods

Test compounds: compound 1 citrate, varenicline tartrate
Reference compound: acetylcholine chloride
Test System: See Example 1.
Oocytes Preparation: See Example 1.
Electrophysiological Recordings: See Example 1.
Agonist Preparation: See Example 1. The test concentrations of compound 1 citrate and varenicline tartrate were 0.1, 0.3, 1, 3, 10, 30, 100, 300 (μM). The test concentration of acetylcholine chloride was 1280 μM.
Data Analysis and Statistics: See Example 1.
Experimental Procedures: Injections of cDNAs encoding for the human alpha3beta4, alpha3alpha5beta4, alpha4-beta2, alpha4alpha6beta2, and alpha7 were performed in at least 95 oocytes using a proprietary automated injection device. Cellular receptor expression was examined at least two days later. Oocytes were poked with two electrodes and their membrane potential maintained, unless indicated, at -80 mV throughout the experiment.

A protocol to determine agonistic activity in the oocytes expressing the nAChRs was developed. Oocytes expressing a nAChR subtype were exposed to a brief test pulse of acetylcholine (1280 μM) while the holding current and response was evaluated. Cells displaying robust currents were washed for 90 seconds. The cells were then exposed to a series of increasing concentrations of a nAChR agonist for 10 seconds each at two minute intervals. The nAChR agonist test concentrations were 0.1, 0.3, 1, 3, 10, 30, 100, 300 (μM). The current evoked from exposure to the agonist at each concentration was recorded. For oocytes expressing alpha7 nAChRs, exposure to the agonist was reduced to 5 seconds. This process was repeated for each agonist tested against each type of nAChR expressing cell.

Results

The effects of compound 1 citrate at human neuronal nicotinic acetylcholine receptors expressed in *Xenopus* oocytes and investigated with two electrode voltage clamp. Determination of the agonistic effects of compound 1 citrate at the alpha3beta4, alpha3alpha5beta4, alpha4beta2, alpha4alpha6beta2, and alpha7 receptors revealed that this compound acts, in the 10 to 300 μM range, as an agonist at these receptors. Noticeable differences were, however, observed in the response between receptor subtypes. Namely, compound 1 citrate was a weak partial agonist at the human alpha7 receptors at a 300 μM concentration. A 300 μM compound 1 citrate solution evoked a response of only 25 percent of the ACh-evoked current. In contrast, data recorded for nAChR subtypes alpha3beta4, alpha3alpha5beta4, alpha4beta2 and alpha4alpha6beta2 revealed that compound 1 citrate acts as an almost full agonist at these receptors.

Compound 1 citrate acts almost as a full agonist at nAChR alpha3beta4, yielding an $EC_{50}$ of 34.87 ± 4.53 μM.

Compound 1 citrate acts almost as a full agonist at nAChR alpha3alpha5beta4, yielding an $EC_{50}$ of 83.00 ± 9.05 μM.

Compound 1 citrate acts almost as a full agonist at nAChR alpha4beta2, yielding an $EC_{50}$ of 13.48 ± 2.06 μM. Significant inhibition of the current evoked by the second Ach exposure is attributed to the desensitization caused by compound 1 citrate exposure.

Compound 1 citrate acts almost as a full agonist at nAChR alpha4alpha6beta2, yielding an $EC_{50}$ of 13.14 ± 3.68 μM.

Compound 1 citrate acts as a poor agonist at nAChR alpha7 evoking at 300 μM only 25 percent of the maximal ACh-evoked current, yielding an $EC_{50}$ of 125.63 ± 28.52 μM. The $EC_{50}$ is an only an estimation given the small fraction of current evoked by compound 1 citrate.

Varenicline acts as weak partial agonist at nAChR alpha4alpha6beta2. At concentrations from 0.1 μM to 300 μM, varenicline evoked a 6% or less response compared to the ACh response. The calculated $EC_{50}$ was 5.02 ± 1.21 μM. Exposure to varenicline was observed to cause a significant inhibition of the subsequent ACh-evoked current.

Example 5 (provided for information only): Pharmacokinetic Measurements in Human Subjects After 28 Days of Intranasal Administration of Compound 1 Hemigalactarate Salt

This example describes a study to determine the plasma systemic exposure and pharmacokinetics after four weeks of treatment with 1.1% Compound 1 (2.0% Hemigalactarate Salt), and after a single bilateral nasal spray on the $29^{th}$ day. The results demonstrate that Compound 1 is detected in all subjects 10 minutes after administration, indicating rapid nasal absorption of Compound 1. In addition, this example demonstrates that Compound 1 exhibits low systemic exposure after intranasal administration and should not exhibit any accumulation in systemic circulation after repeated administration.

Seven patients were administered 1.1% Compound 1 (2.0% hemigalactarate salt) delivered as a 50 μL intranasal spray in each nostril, BID for 28 days. On Day 29, a single intranasal administration of 1.1% Compound 1 (2.0% hemigalactarate salt) was provided to the patients.

Blood plasma for the patients was collected on Day 29 predose (time 0) and after a series of time points (10 min, 20 min, 30 min, 1 hr, 2 hrs, 4 hrs, 5 hrs, 6 hrs, 7 hrs, 8 hrs, and 24 hrs) after a single administration of drug. The blood plasma was analyzed for Compound 1.

The following pharmacokinetic parameters for Compound 1 were calculated:

- **Cmax:** Maximum observed concentration, observed by inspection of individual study participant plasma concentration time plots.
- **Tmax:** Time of maximum observed concentration, obtained directly from the observed concentration time data.
- **AUClast:** The area under the plasma concentration time curve, from time 0 to the last measurable non-zero concentration, calculated by a combination of linear and logarithmic trapezoidal methods (Linear up/log down method).
- **AUC0-4h:** The area under the plasma concentration time curve, from time 0 to the 4 h time point, calculated by a combination of linear and logarithmic trapezoidal methods (Linear up/log down method).
- **AUCinf:** The area from zero time (pre-dose) extrapolated to infinite time (AUCinf) will be calculated as follows: $AUCinf = AUClast + Cz/\lambda z$.
- **AUC%Extrap:** The percentage of AUCinf obtained by extrapolation (AUC%Extrap) will be calculated as follows:

$$AUC\%Extrap = \frac{(AUCinf - AUClast)}{AUCinf} \times 100$$

- **Half-life:** Terminal elimination half-life.

Plasma Concentration Data. After 28 days of BID administration of 1.1% Compound 1, Compound 1 was detected at time zero (predose) in 4 out of 7 subjects with a mean concentration of 0.264 ng/mL. Ten minutes after administration, Compound 1 was detected in plasma from all subjects, indicating rapid nasal absorption. The mean Compound 1 plasma exposure increased with time after intranasal administration with peak of exposure of 2.07 ng/mL at 1 h and then declined through the 24 h sampling period. The decline was biphasic with a plateau between 2 and 4 h. Four of the seven subjects had quantifiable levels at 5 h post dose and there was a loss of one subject with quantifiable Compound 1 at each of the next three time points, 6, 7 and 8 h. There was only one subject with quantifiable Compound 1 8 h post administration and no subjects had quantifiable Compound 1 above the lower limit of quantification of 0.200 ng/mL at 24 h post administration.

Pharmacokinetic Parameter Data. Compound 1 mean (SD) Cmax was 2.26 ± 1.96 ng/mL with an individual subject maximum of 5.49 ng/mL from the seven subjects. The median Tmax occurred at 0.50 h and the mean (SD) half-life was 1.58 ± 0.818 h. The AUC%Extrap in four of the seven subjects was > 20%, which should exclude the calculation of half-life and AUCinf, however, these individual subject values were reported and included in the summary statistics for the PK parameters AUCinf and Half-life. Mean (SD) AUClast was variable at 5.22 ± 4.52 h*ng/mL and the maximum single individual value at 12.1 h*ng/mL. However, one subject with the lowest AUC%Extrap at 3.69% was also the subject with the largest AUClast and AUCinf at 12.1 h*ng/mL and 12.5 h*ng/mL, respectively. The mean (SD) AUCinf was 21.3% greater than mean AUClast at 6.22 ± 4.63 h*ng/mL. The maximum individual subject half-life was 2.83 h, which would suggest that in general if Compound 1 was administered every 12 hours, then > 94% (4 half-lives) of compound 1 would clear from

systemic circulation for all subjects before the next administration. Overall, systemic exposure for Compound 1 was low.

The pharmacokinetic results are summarized in the table below.

| | Half-life (h) | Tmax (h) | Cmax (ng/mL) | AUClast (h*ng/mL) | AUCinf (h*ng/mL) | AUC%Extrap (%) |
|---|---|---|---|---|---|---|
| **N** | 7 | 7 | 7 | 7 | 7 | 7 |
| **Mean** | 1.58 | 0.70 | 2.26 | 5.22 | 6.22 | 21.3 |
| **SD** | 0.818 | 0.38 | 1.96 | 4.52 | 4.63 | 14.6 |
| **Min** | 0.852 | 0.17 | 0.621 | 0.801 | 1.21 | 3.67 |
| **Median** | 1.12 | 0.50 | 1.35 | 4.35 | 5.67 | 23.3 |
| **Max** | 2.83 | 1.2 | 5.49 | 12.1 | 12.5 | 41.1 |

Conclusion. After 28 days of BID administration of 1.1% Compound 1, Compound 1 exhibits low systemic exposure with 4 of 7 subjects (57.1 %), with a mean concentration of 0.264 ng/mL, exhibiting predose systemic exposure. Ten minutes after administration, Compound 1 was detected in plasma from all subjects, indicating rapid nasal absorption. The clearance of Compound 1 was biphasic in nature with a plateau between 2 and 4 h, which may suggest a possible oral component of absorption to the systemic profile. However, there was only one subject with quantifiable amounts of Compound 1 at 8 h post administration and no subjects 24 h post administration. This correlated with the observed mean (SD) half-life of 1.58 ± 0.818 h. The maximum individual subject half-life was 2.83 h, which would suggest that in general if Compound 1 was administered every 12 hours, > 94% (4 half-lives) of Compound 1 would clear from systemic circulation for all subjects before the next administration. Overall, systemic exposure for Compound 1 was low with a mean (SD) Cmax of 2.26 ± 1.96 ng/mL, with an individual subject maximum of 5.49 ng/mL from the seven subjects.

Example 6 (provided for information only): A Phase 2, Multicenter, Randomized, Controlled, Double-Masked, Study to Evaluate the Efficacy of Pre-exposure Prophylaxis Varenicline Tartrate Nasal Spray in the Prevention of SARS-Associated Coronavirus (SARS-CoV-2 [COVID-19]) Infection

The study protocol is summarized below. While the study protocol as drafted includes the administration via a nasal spray, the protocol may be adapted to be used in combination with an inhaler or nebulizer, so that the nAChR agonist reaches the lower respiratory tract. Alternatively, an inhaler or nebulizer may be used instead of the spray. The study protocol may be adapted for the administration of a combination of nAChR agonist and copper. The nAChR agonist and copper may be administered simultaneously in a single pharmaceutical formulation or administered simultaneously in separate dosage formulations. Exemplary concentration ranges of a copper salt administered in a nasal spray, such as copper chloride or copper sulfate, include 1 μM to 500 μM, 1 μM to 100 μM, 1 μM to 50 μM or 1 μM to 10 μM.

| | |
|---|---|
| **Study Objective:** | The objective of this study is to evaluate the safety and effectiveness of A Phase 2, Multicenter, Randomized, Controlled, Double-Masked, Study to Evaluate the Efficacy of Pre-exposure Prophylaxis Varenicline Tartrate Nasal Spray in the Prevention of SARS-Associated Coronavirus (SARS-CoV-2 [COVID-19]) Infection |
| **Investigational Product:** | Varenicline Tartrate nasal spray: 0.6 mg and 1.2 mg |
| **Overall Study Design** | |
| **Structure:** | A Phase 2, multicenter, randomized, controlled, double-masked study |
| **Duration:** | Four (4) study visits over approximately 84 days |
| **Control:** | Placebo (Varenicline Tartrate Vehicle Nasal Spray) |
| **Dosing Regimen:** | Intranasal delivery of Varenicline Tartrate three times daily (TID) for 84 days in one of the following dose groups:<br>• 0.6 mg/mL Varenicline Tartrate nasal spray<br>• 1.2 mg/mL Varenicline Tartrate nasal spray<br>• Placebo nasal spray (vehicle) |

(continued)

| Overall Study Design | |
|---|---|
| **Summary of Visit Schedule:** | **Visit 1-** Screening and randomization (Day 1)<br>**Visit 2-** SARS-CoV-2 Testing and Symptom assessment (Day 28)<br>**Visit 3-** SARS-CoV-2 Testing and Symptom assessment (Day 56)<br>**Visit 4-** SARS-CoV-2 Testing and Symptom assessment (Day 84) |
| **Measures Taken to Reduce Bias:** | This is a randomized, double-masked study |
| **Study Population Characteristics** | |
| **Number of Subjects:** | Approximately 600 (200 per arm) |
| **Condition/Disease:** | SARS-Associated Coronavirus (SARS-CoV-2 [COVID-19]) Infection |

The inclusion criteria are summarized below.

| | |
|---|---|
| **Inclusion Criteria:** | **Subjects must:**<br>1. Have provided written and verbal informed consent<br>2. Be at least 18 years of age at the Screening Visit<br>3. Not be not currently or previously diagnosed with COVID-19<br>4. Not be symptomatic with an Acute Respiratory Infection<br>5. Work as a healthcare worker or frontline (i.e. patient contact) in a healthcare facility or similar institution.<br>6. Be willing to participate in the trial and can be followed adequately for up to 3 months.<br>7. Participant is willing and able to give informed consent for participation in the study and agrees with the study and its conduct.<br>8. Agree not to self-medicate with other potential antivirals or therapies. |

The exclusion criteria are summarized below

| | |
|---|---|
| **Exclusion Criteria:** | **Subjects must not:**<br>1. Have a history of seizures or other factors that lower the subject's seizure threshold.<br>2. Have a systemic condition or disease not stabilized or judged by the Investigator to be incompatible with participation in the study or with the lengthier assessments required by the study (e.g., current systemic infection, uncontrolled autoimmune disease, uncontrolled immunodeficiency disease, history of myocardial infarction or heart disease, etc.)<br>3. Have a known hypersensitivity to any of the procedural agents or study drug components<br>4. Have current concomitant use of a nicotinic acetylcholine receptor agonist [Nicoderm®, Nicorette®, Nicotrol NS® (nicotine), Tabex®, Desmoxan® (cytisine), and Chantix® (varenicline)] or within the previous 30 days<br>5. Have a documented concomitant severe bacterial or fungal infection with HIV, HCV, HBV<br>6. Have any condition or history that, in the opinion of the investigator, may interfere with study compliance, outcome measures, safety parameters, and/or the general medical condition of the subject<br>7. Be a female who is pregnant, nursing an infant, or planning a pregnancy at Visit 1. Be a woman of childbearing potential who is not using an acceptable means of birth control; acceptable methods of contraception include: hormonal - oral, implantable, injectable, or transdermal contraceptives; mechanical- spermicide in conjunction with a barrier such as a diaphragm or condom; IUD; or surgical sterilization of partner.<br>8. Be currently enrolled in an investigational drug or device study or have used an investigational drug or device within 30 days prior to Visit 1 |

The study protocol is summarized below.

| | |
|---|---|
| **Study Formulations:** | Subjects will be randomized 1:1:1 to be treated with Varenicline Tartrate nasal solution delivered as a 50 μL intranasal spray in each nostril at the following formulations:<br>• 0.6 mg/mL Varenicline Tartrate nasal spray<br>• 1.2 mg/mL Varenicline Tartrate nasal spray<br>• Placebo nasal spray (Varenicline Tartrate Vehicle Nasal Spray) |
| **Evaluation Criteria** | |
| **Efficacy Measures:** | • Number of symptomatic COVID-19 infections will be compared between the 0.6 mg and 1.2 mg and placebo groups at Day 28, Day 56, and Day 84<br>• Number of asymptomatic COVID-19 infections will be compared between the 0.6 mg and 1.2 mg and placebo groups at Day 28, Day 56, and Day 84<br>• Severity of symptoms COVID-19 infections will be compared between the 0.6 mg and 1.2 mg and placebo groups at Day 28, Day 56, and Day 84 categorized as:<br>• No symptoms<br>• Mild symptoms: general malaise, fever, cough, myalgia, asthenia.<br>• Moderate symptoms: mild symptoms plus shortness of breath,<br>• Severe symptoms: mild symptoms plus respiratory insufficiency that requires admission in intensive care unit and mechanical ventilation |
| **Safety Measures:** | • Adverse Event (AE) Query |

A schedule of proposed visits and measurements is summarized below.

| Procedure | Screen/Visit 1 Day 1 | Visit 2 Week 4 Day 28 ±3 | Visit 3 Week 8 Day 56 ±3 | Visit 4 Week12 Day 84 ±3 | ET |
|---|---|---|---|---|---|
| Informed consent/HIPAA | X | | | | |

(continued)

| **Procedure** | **Screen/Visit 1 Day 1** | **Visit 2 Week 4 Day 28 ±3** | **Visit 3 Week 8 Day 56 ±3** | **Visit 4 Week12 Day 84 ±3** | **ET** |
|---|---|---|---|---|---|
| Demographics | X | | | | |
| Medical history, | X | | | | |
| prior medication(s), and updates | | | | | |
| Eligibility criteria | X | | | | |
| Urine pregnancy test | X1 | | | | X1 |
| Intranasal examination | X | | | X | X |
| Randomization | X | | | | |
| SARS-CoV Testing | X | X | X | X | X |
| Dispense investigational drug / placebo | X | X | X | X | |
| Symptom Assessment | X | X | X | X | X |
| Concomitant medications | X | X | X | X | X |
| AE Query | X | X | X | X | X |
| X1 = For females of childbearing potential; ET=Early Termination | | | | | |

Example 7: Antiviral activity of Varenicline and Copper Chloride in Caco-2 cells after exposure to SARS-CoV-2

The objective of this study was to evaluate the protective effect of varenicline tartrate, copper chloride, or the combination thereof on Caco-2 cells from infection with SARS-CoV-2. Caco-2 cells were pre-treated with varenicline tartrate and/or copper chloride for 1 hour. Simultaneously, SARS-CoV-2 was also pre-treated with varenicline tartrate and/or copper chloride for 1 hour. The cell and viral pre-treated mixtures were combined for 2 hours. Next, the cells were washed, and fresh varenicline tartrate and/or copper chloride solution was added to the cells. After 24 hours from exposure to the virus, the resulting cell culture supernatants were harvested and the amount of virus in the supernatant was measured using plaque titration. Based on the results, the percent infection of Caco-2 cells with SARS-CoV-2 is calculated. These numbers are normalized based on the infection rates of cells not treated with varenicline tartrate and/or copper chloride.

In parallel, cell viability in the presence of varenicline tartrate and/or copper chloride was determined using CellTiter-Glo assay (Promega), which measures intracellular adenosine triphosphate levels. The Caco-2 cells were treated with respective concentration of varenicline tartrate and/or copper chloride for 72 h and evaluated with the CellTiter-Glo assay, where luminescence was measured.

The data for % infection efficiency and % cell viability was normalized against the data from a control study where varenicline tartrate and/or copper chloride was absent.

**Experimental Method and Calculation of Infection of Caco-2 Cells After Exposure to SARS-CoV-2**

*Preparation of viral and therapeutic agent solutions.* SARS-CoV-2 wild-type clinical isolate, USA-WA1/2020 was obtained from BEI and passaged in Vero E6 cells at Trudeau Institute to create working virus stocks. The virus working stock used in the study was passaged two times in Vero E6 cells prior to use. Working stocks of SARS-CoV-2 were diluted to generate a multiplicity of infection (MOI) of 3 in Caco-2 cells, i.e. three viral particles per cell.

A series of dilutions of varenicline tartrate and copper chloride dihydrate was also prepared.

*Pre-treatment with varenicline tartrate and/or copper chloride for 1 h.* Caco-2 cells were pre-treated with varenicline tartrate and/or copper chloride hydrate at various dilutions for 1h. Separately, a solution of SARS-CoV-2 (20 μL) was pretreated with 180 μL of varenicline tartrate and/or cupric chloride hydrate for 1h at 37° C and under 5% $CO_2$. In the control study, varenicline tartrate and/or copper chloride solution is absent and DMSO is used instead.

*Exposure of Caco-2 cells to viral solution for 2 h.* Following the 1 h pre-treatment period, the pre-treated Caco-2 cells were exposed to 50 ml of the pre-treated SARS-CoV-2 for 2 h with regular shaking of plate every 15 minutes. After the 2 h exposure, the culture supernatant was removed and the remaining Caco-2 cells were washed twice with PBS. Fresh culture medium containing varenicline tartrate and/or copper chloride dihydrate was then added to the Caco-2 cells. In the control experiment, DMSO was added.

*Calculation of viral particles via plaque titration 24 hours after exposure.* Cell culture supernatants were harvested at 24 h post SARS-CoV-2 exposure and subjected to plaque titration. For plaque titration, confluent Vero-E6 cells were inoculated with 250 ml of 5-fold serial dilutions of Caco-2 culture supernatant and incubated for 1 h at 37 °C and 5% CO2. Following this incubation period, 750 ml of culture medium containing 2.4% (final concentration 0.6%) microcrystalline cellulose was added to the cultures. Plaques were counted at 72 h post infection and titers determined as plaque forming units per ml (pfu/ml).

The percent infection of Caco-2 cells was normalized against samples untreated with varenicline tartrate and/or copper chloride. Error bars represent mean and standard deviation of three independent replicates. The experiment was performed in triplicate.

**Experimental Method and Calculation of Caco-2 Cell Viability After 72 Hours Of Exposure to Varenicline Tartrate and/or Copper Chloride**

Caco-2 cells were plated in white, opaque-walled 96-well plates at 1,000 cells/well and treated with a 7-point dose response curve with 3-fold serial dilution of varenicline tartrate and copper chloride dihydrate. Cell viability was determined 72 hours after varenicline tartrate and/or cupric chloride dihydrate treatment using the CellTiter-Glo Luminescent Cell Viability Assay kit (Promega). Cells treated with DMSO (solvent control) served as controls. Following incubation, 10 ml of CellTiter-Glo substrate was added to each well and the samples were incubated for 10 min on a rocking platform. Following this 10 min incubation, luminescence was recorded using a POLARstar OPTIMA plate reader (BMG Labtech). Samples were run in triplicate and data is normalized with mean luminescence of DMSO only treated cells as 100% viability.

**Statistical analyses**

All statistical analyses were performed using GraphPad Prism (version 9, GraphPad Software, Inc.). For the calculation of 50% inhibitory concentration ($IC_{50}$) and 50% cytotoxic concentration ($CC_{50}$) of the compound which indicate the inhibitor concentration leading to 50% reduction of infection or cell viability respectively, non-linear fit regression models were used.

**Dose-dependent inhibition of SARS-CoV-2 human epithelial cell infection by varenicline**

This example describes the effect of varenicline tartrate on infection rates of Caco-2 cells 24 hours after exposure to SARS-CoV-2. Using the methods described in the protocol above, dilutions of varenicline tartrate were prepared and the Caco-2 cells were exposed to increasing concentrations of varenicline tartrate at 0.1 μM, 0.4 μM, 1.2 μM, 3.7 μM, 11.1 μM, 33.3 μM, and 100 μM. FIG. 3 shows a dose dependent decrease in the percentage of Caco-2 cells infected with SARS-CoV-2 (left y-axis) over a 24 hour period using a multiplicity of infection (MOI) of 3 (viral particles per cell) as compared to the vehicle control. The results indicate a dose-dependent decrease in the percentage of Caco-2 cells infected with virus with no significant impact on cellular viability as assessed at 72 hours post-drug exposure (right y-axis), even at 100 μM varenicline tartrate.

**Dose-dependent inhibition of SARS-CoV-2 human epithelial cell infection by copper chloride**

This example describes the effect of copper chloride on infection rates of Caco-2 cells 24 hours after exposure to SARS-CoV-2. Using the methods described in the protocol above, dilutions of copper chloride were prepared and the Caco-2 cells were exposed to increasing concentrations of copper chloride at 0.003 μM, 0.008 μM, 0.02 μM, 0.07 μM, 0.22 μM, 0.67 μM, and 2 μM. FIG. 4 shows a dose dependent decrease in the percentage of Caco-2 cells infected with SARS-CoV-2 over a 24 hour period using a multiplicity of infection (MOI) of 3 (viral particles per cell) as compared to the vehicle control. The results support a dose-dependent decrease in the percentage of Caco-2 cells infected with virus with no significant impact on cellular viability as assessed at 72 hours post-drug exposure at copper chloride concentrations of less than 1 μM. Some cellular toxicity is evident at exposures greater than 1 μM copper chloride.

**At Lower Varenicline Tartrate Concentrations, the Combination of Varenicline Tartrate and Copper Chloride Demonstrates a Greater Inhibition of Sars-Cov-2 Infection of Epithelial Cells Compared to Varenicline Tartrate Alone**

This example describes the combined effect of varenicline tartrate and 0.3 μM copper chloride on infection rates of Caco-2 cells 24 hours after exposure to SARS-CoV-2. Using the methods described in the protocol above, dilutions of varenicline tartrate were prepared and the Caco-2 cells were exposed to increasing concentrations of varenicline tartrate at 0.1 μM, 0.4 μM, 1.2 μM, 3.7 μM, 11.1 μM, 33.3 μM, and 100 μM, and 0.3 μM copper chloride. FIG. 5 shows the percent

infection rates, left y-axis, after 24 hours from exposure to the virus as a function of varenicline tartrate concentration. The right y-axis shows the percent viability of the cells after 72 hours as a function of varenicline tartrate concentration. At the lower concentrations of varenicline tartrate (0.1 μM and 0.5 μM), the infection rate of the cells was approximately 25% when in the presence of 0.3 μM copper chloride. In comparison, the infection rate at the same varenicline tartrate concentrations without copper chloride was closer to 100% as shown in FIG. 3. Thus, greater inhibition of the virus in cells is achieved at the lower tested concentrations of varenicline tartrate in the presence of 0.3 μM copper chloride compared to varenicline alone.

Furthermore, at exposures of less than 50 μM varenicline tartrate/0.3 μM copper chloride, no significant impact on cellular viability is observed. At exposures of greater than 50 μM varenicline tartrate/0.3 μM copper chloride, some cellular toxicity is evident.

Example 8: A 5-Day QID Nasal Installation Study of Varenicline Tartrate and Varenicline Tartrate/Copper Chloride in Non-Human Primates

The objective of this experiment is to evaluate the pre-exposure prophylaxis administration of varenicline tartrate and/or the combination of varenicline tartrate/copper chloride nasal spray on SARS-CoV-2 challenge in non-human primates. Test articles comprising varenicline tartrate and/or the combination of varenicline tartrate/copper chloride have been generated to prevent the cellular entry of SARS-CoV-2 and permanently inactive the virus from further infection, replication or shedding. The administration of test articles will be in the form of a nasal spray, which is a primary route of host entry for SARS-CoV-2. This study will utilize a short prophylactic treatment period followed by infecting non-human primates with SARS-CoV-2 and then additional days of therapeutic dosing with a nasal spray administered four times per day in each nostril.

Each animal will be treated with a total of 100 μL of nasal spray of test article into each nostril four times daily for 5 days.

**Day 1**

On Day 1, the first and second daily doses of test article(s) will be administered. After 2$^{nd}$ dose, animals will be challenged with SARS-CoV-2 0.1 ml (100 μL)/nostril (1x10^5 PFU of virus). Finally, the third and fourth daily doses of test article will be administered on Day 1.

**Day 2, 3, 4, 5, 6 (Day 6: 2 doses prior to study termination).**

The test article will be administered into each nostril (naris) four times daily for 5 days. Animals will be sacrificed on Day 6 (5 days from virus challenge).

**STUDY DESIGN/DOSING**

| Group No. | Test Material | Dose Concentration | Total Dose Volume (μL, 2 naris) | Number of Animals |
|---|---|---|---|---|
| 1 | varenicline tartrate | 0.6 mg/ml | 200 | 2 |
| 2 | varenicline tartrate | 1.2 mg/ml | 100 | 2 |
| | copper chloride | 3 μM | 100 | |

The test materials will be intranasally administered via an aqueous nasal spray. The dose frequency of the test material is four times daily in each naris, as specified above over 6 Days.

Group No. 1 will be dosed with two 50 μL sprays of varenicline tartrate nasal spray (0.6 mg/ml) into each naris at each dosing occasion for a total dose volume of 200 μL.

Group No. 2 will be dosed with a single 50 μL spray to each naris of varenicline tartrate nasal spray (1.2 mg/ml) and a single 50 μL spray to each naris of copper chloride (3 μM $CuCl_2$). The time between the administration of varenicline tartrate and copper chloride is about 1 minute.

## BIOANALYTICAL

RT-PCR. Nasal swabs will be collected and used to measure virus genomic and subgenomic virus RNA) on days 2, 4 and 6. Bronchoalveolar lavage (BAL) fluid will be collected and analyzed on days 2, 4 and 6.

Plaque Assay. Nasal swabs will be collected on days 2, 4 and 6 and subjected to plaque assay. Bronchoalveolar lavage (BAL) fluid will be collected and analyzed on day 6.

## PATHOLOGY

A necropsy of all animals will be carried out and the organ weights analyzed (standard panel). Histopathology to be performed on standard tissue list in control and high dose animals, with read-down for findings. Nares to be fixed in formalin at Day 8 for chromogenic in situ hybridization (CISH) and SARS-CoV-2 immunohistochemical analysis (IHC) in serial tissue sections. Lung to be fixed in formalin at Day 8 for chromogenic in situ hybridization (CISH) and SARS-CoV-2 immunohistochemical analysis (IHC) in serial tissue sections.

While embodiments of the present invention have been shown and described herein, those skilled in the art will understand that such embodiments are provided by way of example only. It should be understood that various alternatives to the embodiments of the invention described herein may be employed in practicing the invention. It is intended that the following claims define the scope of the invention.

## Claims

**1.** A compound for use in a method of inhibiting a coronavirus infection in an individual in need thereof, wherein the compound is a nAChR agonist, or a pharmaceutically acceptable salt thereof, wherein the nAChR agonist is varenicline, wherein the method comprises locally administering the nAChR agonist, or a pharmaceutically acceptable salt thereof, into the respiratory tract of the individual in need thereof, and wherein the method further comprises locally administering a pharmaceutically acceptable salt of copper into the respiratory tract of the individual in need thereof.

**2.** The compound for use of claim 1, wherein the coronavirus is SARS-CoV-2 or SARS-CoV-1.

**3.** The compound for use of claim 1 or claim 2, wherein the local administration is to the upper respiratory tract, or

wherein the local administration is to the nasal cavity, or
wherein the local administration is to the pharynx, bronchi, lungs, oral mucosa, or a combination of the foregoing, or
wherein the local administration is to the lower respiratory tract, or
wherein the local administration is to both the upper and lower respiratory tract.

**4.** The compound for use of any one of the preceding claims, wherein administration into the nasal cavity is via an nasal spray or nasal nebulizer, and/or wherein local administration is via an inhaler or nebulizer.

**5.** The compound for use of any one of the preceding claims, wherein 5-4000 $\mu$g of the nAChR agonist, or a corresponding amount of a pharmaceutically acceptable salt thereof, per dose is administered to the individual.

**6.** The compound for use of any one of the preceding claims, wherein the dose of the nAChR agonist, or a pharmaceutically acceptable salt thereof, is administered in a pharmaceutical formulation for local respiratory administration, and the total volume of the pharmaceutical formulation administered per dose of the nAChR agonist, or a pharmaceutically acceptable salt thereof, to the individual is 50 $\mu$L-250 $\mu$L, or
wherein the dose of the nAChR agonist, or a pharmaceutically acceptable salt thereof, is administered in a pharmaceutical formulation for nasal administration, and the total volume of the pharmaceutical formulation administered per nostril of the nAChR agonist, or a pharmaceutically acceptable salt thereof, to the individual is 50 $\mu$L-250 $\mu$L.

**7.** The compound for use of any one of the preceding claims, wherein the nAChR agonist, or a pharmaceutically acceptable salt thereof, is administered in a pharmaceutical formulation, wherein the formulation is a liquid, suspension, aerosol, gel, ointment, dry powder, cream, paste, balm, or nasal spray, and/or
wherein the nAChR agonist, or a pharmaceutically acceptable salt thereof, is administered in a pharmaceutical

formulation administered into the nasal cavity by a syringe, dropper, bottle nebulizer, atomization pump, inhaler, powder spray device, vaporizer, medicated stick, pipette, jet of liquid, or nasal spray bottle.

**8.** The compound for use of any one of the preceding claims, wherein copper chloride or copper sulfate is administered to the individual in need thereof.

**9.** The compound for use of any one of the preceding claims, wherein the nAChR agonist, or a pharmaceutically acceptable salt thereof, and the pharmaceutically acceptable salt of copper, is administered separately to the individual in need thereof, or
wherein the nAChR agonist, or a pharmaceutically acceptable salt thereof, and the pharmaceutically acceptable salt of copper, is administered in a combined pharmaceutical formulation to the individual in need thereof.

**10.** The compound for use of any one of the preceding claims, wherein the pharmaceutically acceptable salt of copper is administered to the individual in need thereof in a pharmaceutical formulation comprising a concentration of the pharmaceutically acceptable salt of copper corresponding to a concentration of between 0.001 μM and 500 μM of copper.

**11.** A pharmaceutical formulation comprising a nicotinic acetylcholine receptor (nAChR) agonist and a pharmaceutically acceptable salt of copper, wherein the nAChR agonist is varenicline, or a pharmaceutically acceptable salt thereof.

**12.** The pharmaceutical formulation of claim 11, comprising copper chloride or copper sulfate.

**13.** The pharmaceutical formulation of claim 11 or claim 12, comprising a concentration of a pharmaceutically acceptable salt of copper corresponding to a concentration of between 0.001 μM and 500 μM of copper, and wherein the nAChR agonist is at a concentration of between 1 mg/mL and 40 mg/mL, or a corresponding amount of a pharmaceutically acceptable salt thereof.

**14.** The pharmaceutical formulation of any one of claims 11-13, wherein the pharmaceutical formulation is a liquid.

**15.** The compound for use of any one of claims 1-10, or the pharmaceutical formulation of any one of claims 11-14, wherein the nAChR agonist is varenicline tartrate.

## Patentansprüche

**1.** Verbindung zur Verwendung in einem Verfahren zur Inhibierung einer Coronavirusinfektion in einem Individuum, das dessen bedarf, wobei die Verbindung ein nAChR-Agonist ist, oder ein pharmazeutisch verträgliches Salz davon, wobei der nAChR-Agonist Vareniclin ist, wobei das Verfahren die lokale Verabreichung des nAChR-Agonisten umfasst, oder eines pharmazeutisch verträglichen Salzes davon, in die Atemwege des Individuums, das dessen bedarf, umfasst und wobei das Verfahren ferner die lokale Verabreichung eines pharmazeutisch verträglichen Kupfersalzes in die Atemwege des Individuums, das dessen bedarf, umfasst.

**2.** Verbindung zur Verwendung nach Anspruch 1, wobei das Coronavirus SARS-CoV-2 oder SARS-CoV-1 ist.

**3.** Verbindung zur Verwendung nach Anspruch 1 oder Anspruch 2, wobei die lokale Verabreichung in die oberen Atemwege erfolgt, oder

wobei die lokale Verabreichung in die Nasenhöhle erfolgt, oder
wobei die lokale Verabreichung in den Rachen, in die Bronchien, in die Lunge, in die Mundschleimhaut oder in eine Kombination der vorgenannten erfolgt, oder
wobei die lokale Verabreichung in die unteren Atemwege erfolgt, oder
wobei die lokale Verabreichung sowohl in die oberen als auch in die unteren Atemwege erfolgt.

**4.** Verbindung zur Verwendung nach einem der vorhergehenden Ansprüche, wobei die Verabreichung in die Nasenhöhle über ein Nasenspray oder einen Nasenvernebler erfolgt und/oder wobei die lokale Verabreichung über einen Inhalator oder Vernebler erfolgt.

**5.** Verbindung zur Verwendung nach einem der vorhergehenden Ansprüche, wobei 5 μg bis 4000 μg des nAChR-

Agonisten oder eine entsprechende Menge eines pharmazeutisch verträglichen Salzes davon pro Dosis an das Individuum verabreicht wird.

6. Verbindung zur Verwendung nach einem der vorhergehenden Ansprüche, wobei die Dosis des nAChR-Agonisten oder eines pharmazeutisch verträglichen Salzes davon in einer pharmazeutischen Formulierung zur lokalen Verabreichung über die Atemwege verabreicht wird und das Gesamtvolumen der pharmazeutischen Formulierung, die dem Individuum pro Dosis des nAChR-Agonisten oder eines pharmazeutisch verträglichen Salzes davon verabreicht wird, 50 µl bis 250 µl beträgt, oder
wobei die Dosis des nAChR-Agonisten oder eines pharmazeutisch verträglichen Salzes davon in einer pharmazeutischen Formulierung zur nasalen Verabreichung verabreicht wird und das Gesamtvolumen der pharmazeutischen Formulierung des nAChR-Agonisten oder eines pharmazeutisch verträglichen Salzes davon, die dem Individuum pro Nasenloch verabreicht wird, 50 µl bis 250 µl beträgt.

7. Verbindung zur Verwendung nach einem der vorhergehenden Ansprüche, wobei der nAChR-Agonist oder ein pharmazeutisch verträgliches Salz davon in einer pharmazeutischen Formulierung verabreicht wird, wobei die Formulierung eine Flüssigkeit, eine Suspension, ein Aerosol, ein Gel, eine Salbe, ein Trockenpulver, eine Creme, eine Paste, ein Balsam oder ein Nasenspray ist, und/oder
wobei der nAChR-Agonist oder ein pharmazeutisch verträgliches Salz davon in einer pharmazeutischen Formulierung verabreicht wird, die durch eine Spritze, einen Tropfer, einen Flaschenvernebler, eine Zerstäuberpumpe, einen Inhalator, eine Pulversprühvorrichtung, einen Verdampfer, einen Medikamentenstab, eine Pipette, einen Flüssigkeitsstrahl oder eine Nasensprühflasche in die Nasenhöhle verabreicht wird.

8. Verbindung zur Verwendung nach einem der vorhergehenden Ansprüche, wobei Kupferchlorid oder Kupfersulfat dem Individuum, das dessen bedarf, verabreicht wird.

9. Verbindung zur Verwendung nach einem der vorhergehenden Ansprüche, wobei der nAChR-Agonist oder ein pharmazeutisch verträgliches Salz davon und das pharmazeutisch verträgliche Kupfersalz dem Individuum, das dessen bedarf, getrennt verabreicht wird, oder
wobei der nAChR-Agonist oder ein pharmazeutisch verträgliches Salz davon und das pharmazeutisch verträgliche Kupfersalz in einer kombinierten pharmazeutischen Formulierung an das Individuum, das dessen bedarf, verabreicht wird.

10. Verbindung zur Verwendung nach einem der vorhergehenden Ansprüche, wobei das pharmazeutisch verträgliche Kupfersalz dem Individuum, das dessen bedarf, in einer pharmazeutischen Formulierung verabreicht wird, die eine Konzentration des pharmazeutisch verträglichen Kupfersalzes umfasst, die einer Konzentration zwischen 0,001 µM und 500 µM Kupfer entspricht.

11. Pharmazeutische Formulierung, umfassend einen nikotinischen Acetylcholinrezeptor(nAChR)-Agonisten und ein pharmazeutisch verträgliches Kupfersalz, wobei der nAChR-Agonist Vareniclin oder ein pharmazeutisch verträgliches Salz davon ist.

12. Pharmazeutische Formulierung nach Anspruch 11, umfassend Kupferchlorid oder Kupfersulfat.

13. Pharmazeutische Formulierung nach Anspruch 11 oder Anspruch 12, umfassend eine Konzentration eines pharmazeutisch verträglichen Kupfersalzes, die einer Konzentration zwischen 0,001 µM und 500 µM Kupfer entspricht, und wobei der nAChR-Agonist in einer Konzentration zwischen 1 mg/ml und 40 mg/ml vorliegt, oder eine entsprechende Menge eines pharmazeutisch verträglichen Salzes davon.

14. Pharmazeutische Formulierung nach einem der Ansprüche 11 bis 13, wobei die pharmazeutische Formulierung eine Flüssigkeit ist.

15. Verbindung zur Verwendung nach einem der Ansprüche 1 bis 10 oder die pharmazeutische Formulierung nach einem der Ansprüche 11 bis 14, wobei der nAChR-Agonist Vareniclin-Tartrat ist.

## Revendications

1. Composé destiné à être utilisé dans un procédé d’inhibition d’une infection à coronavirus chez un individu qui en a

besoin, dans lequel le composé est un agoniste de nAChR, ou un sel pharmaceutiquement acceptable de celui-ci, dans lequel l'agoniste de nAChR est la varénicline, dans lequel le procédé comprend l'administration locale de l'agoniste de nAChR, ou d'un sel pharmaceutiquement acceptable de celui-ci, dans les voies respiratoires de l'individu qui en a besoin, et dans lequel le procédé comprend en outre l'administration locale d'un sel de cuivre pharmaceutiquement acceptable dans les voies respiratoires de l'individu qui en a besoin.

**2.** Composé destiné à être utilisé selon la revendication 1, dans lequel le coronavirus est le SARS-CoV-2 ou le SARS-CoV-1.

**3.** Composé destiné à être utilisé selon la revendication 1 ou la revendication 2, dans lequel l'administration locale se fait dans les voies respiratoires supérieures, ou

dans lequel l'administration locale se fait dans la cavité nasale, ou
dans lequel l'administration locale se fait dans le pharynx, le bronches, les poumons, la muqueuse buccale ou à une combinaison des précédents, ou
dans lequel l'administration locale se fait dans les voies respiratoires inférieures, ou
dans lequel l'administration locale se fait à la fois dans les voies respiratoires supérieures et inférieures.

**4.** Composé destiné à être utilisé selon l'une quelconque des revendications précédentes, dans lequel l'administration dans la cavité nasale se fait via un pulvérisateur nasal ou un nébuliseur nasal, et/ou dans lequel l'administration locale se fait via un inhalateur ou un nébuliseur.

**5.** Composé destiné à être utilisé selon l'une quelconque des revendications précédentes, dans lequel 5-4000 μg de l'agoniste de nAChR, ou une quantité correspondante d'un sel pharmaceutiquement acceptable de celui-ci, par dose sont administrés à l'individu.

**6.** Composé pour l'utilisation selon l'une quelconque des revendications précédentes, dans lequel la dose de l'agoniste de nAChR, ou d'un sel pharmaceutiquement acceptable de celui-ci, est administrée dans une formulation pharmaceutique pour une administration respiratoire locale, et le volume total de la formulation pharmaceutique, administrée par dose à l'individu, de l'agoniste de nAChR, ou d'un sel pharmaceutiquement acceptable de celui-ci, est de 50 μL-250 μL, ou
dans lequel la dose de l'agoniste de nAChR, ou d'un sel pharmaceutiquement acceptable de celui-ci, est administrée dans une formulation pharmaceutique pour administration nasale, et le volume total de la formulation pharmaceutique, administrée à l'individu par la narine, de l'agoniste de nAChR, ou d'un sel pharmaceutiquement acceptable de celui-ci, est de 50 μL-250 μL.

**7.** Composé destiné à être utilisé selon l'une quelconque des revendications précédentes, dans lequel l'agoniste de nAChR, ou un sel pharmaceutiquement acceptable de celui-ci, est administré dans une formulation pharmaceutique, dans lequel la formulation est un liquide, une suspension, un aérosol, un gel, un onguent, une poudre sèche, une crème, une pâte, un baume ou une pulvérisation nasale, et/ou
dans lequel l'agoniste de nAChR, ou un sel pharmaceutiquement acceptable de celui-ci, est administré dans une formulation pharmaceutique administrée dans la cavité nasale par une seringue, un compte-gouttes, un nébuliseur à flacon, une pompe d'atomisation, un inhalateur, un dispositif de pulvérisation de poudre, un vaporisateur, un bâtonnet médicamenteux, une pipette, un jet de liquide ou un flacon de pulvérisation nasale.

**8.** Composé destiné à être utilisé selon l'une quelconque des revendications précédentes, dans lequel du chlorure de cuivre ou du sulfate de cuivre est administré à l'individu qui en a besoin.

**9.** Composé destiné à être utilisé selon l'une quelconque des revendications précédentes, dans lequel l'agoniste de nAChR, ou un sel pharmaceutiquement acceptable de celui-ci, et le sel de cuivre pharmaceutiquement acceptable, sont administrés séparément à l'individu qui en a besoin, ou
dans lequel l'agoniste de nAChR, ou un sel pharmaceutiquement acceptable de celui-ci, et le sel de cuivre pharmaceutiquement acceptable, sont administrés dans une formulation pharmaceutique combinée à l'individu qui en a besoin.

**10.** Composé destiné à être utilisé selon l'une quelconque des revendications précédentes, dans lequel le sel de cuivre pharmaceutiquement acceptable est administré à l'individu qui en a besoin dans une formulation pharmaceutique comprenant une concentration du sel de cuivre pharmaceutiquement acceptable correspondant à une concentration

d'entre 0,001 μM et 500 μM de cuivre.

**11.** Formulation pharmaceutique comprenant un agoniste du récepteur nicotinique de l'acétylcholine (nAChR) et un sel de cuivre pharmaceutiquement acceptable, dans laquelle l'agoniste de nAChR est la varénicline, ou un sel pharmaceutiquement acceptable de celle-ci.

**12.** Formulation pharmaceutique selon la revendication 11, comprenant du chlorure de cuivre ou du sulfate de cuivre.

**13.** Formulation pharmaceutique selon la revendication 11 ou la revendication 12, comprenant une concentration d'un sel de cuivre pharmaceutiquement acceptable correspondant à une concentration d'entre 0,001 μM et 500 μM de cuivre, et dans laquelle l'agoniste de nAChR est à une concentration d'entre 1 mg/mL et 40 mg/mL, ou une quantité correspondante d'un sel pharmaceutiquement acceptable de celui-ci.

**14.** Formulation pharmaceutique selon l'une quelconque des revendications 11 à 13, dans laquelle la formulation pharmaceutique est un liquide.

**15.** Composé destiné à être utilisé selon l'une quelconque des revendications 1 à 10, ou formulation pharmaceutique selon l'une quelconque des revendications 11 à 14, dans lesquels l'agoniste de nAChR est le tartrate de varénicline.

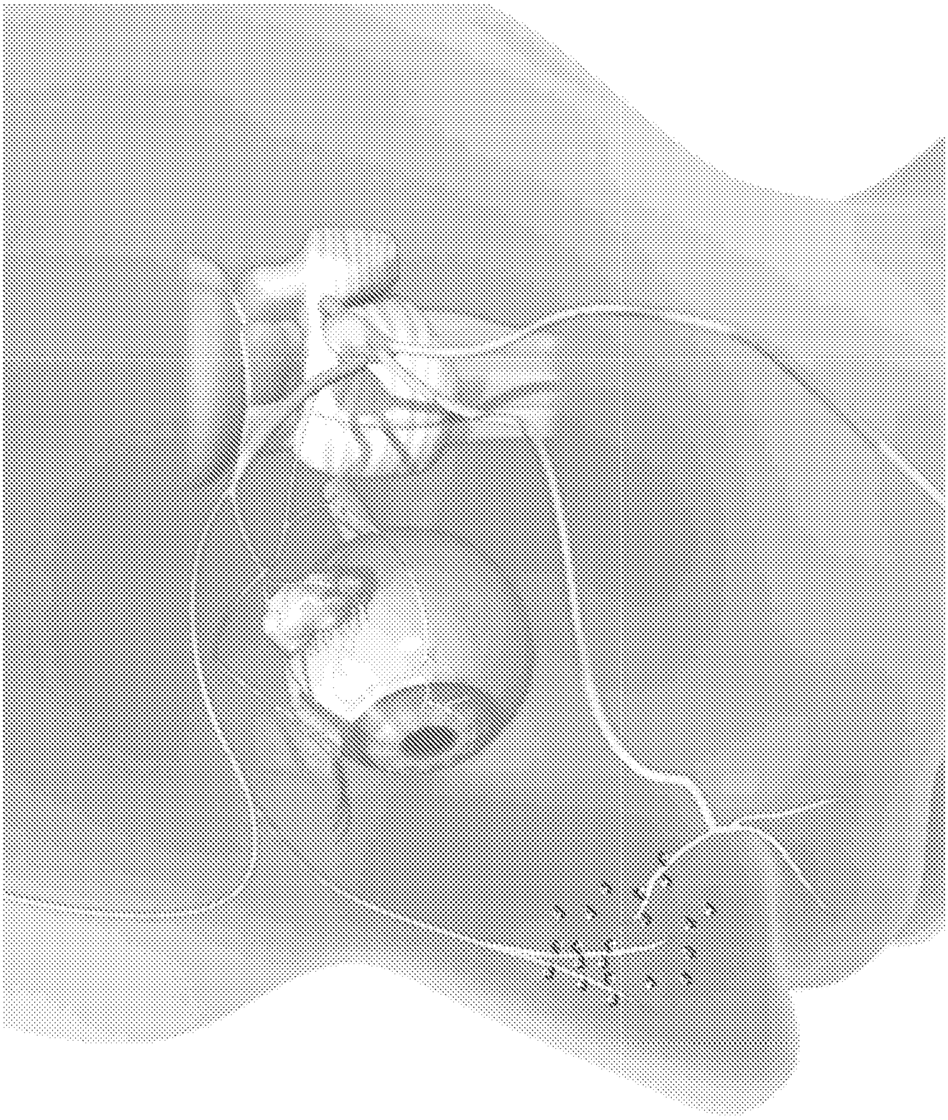

FIG. 1

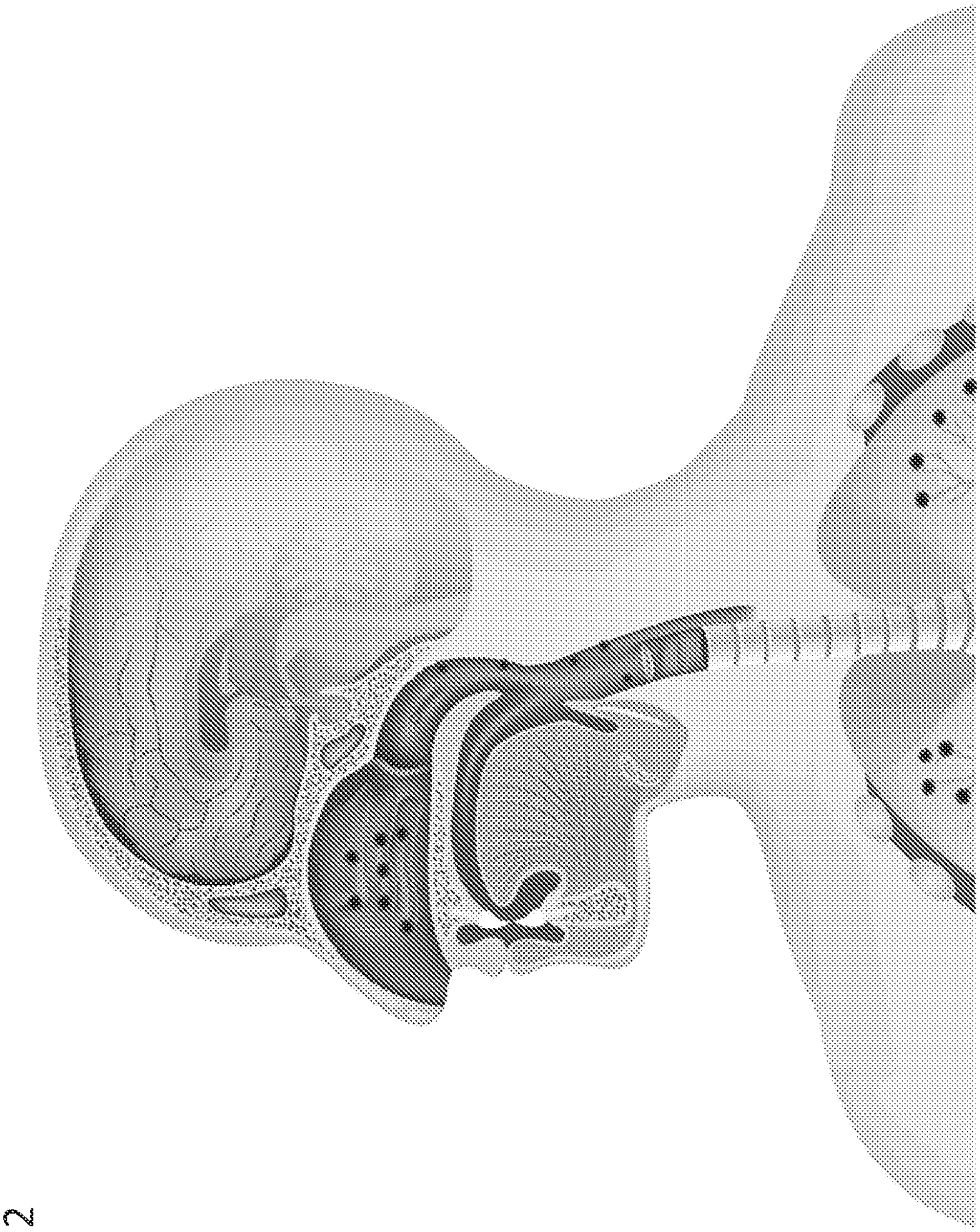

FIG. 2

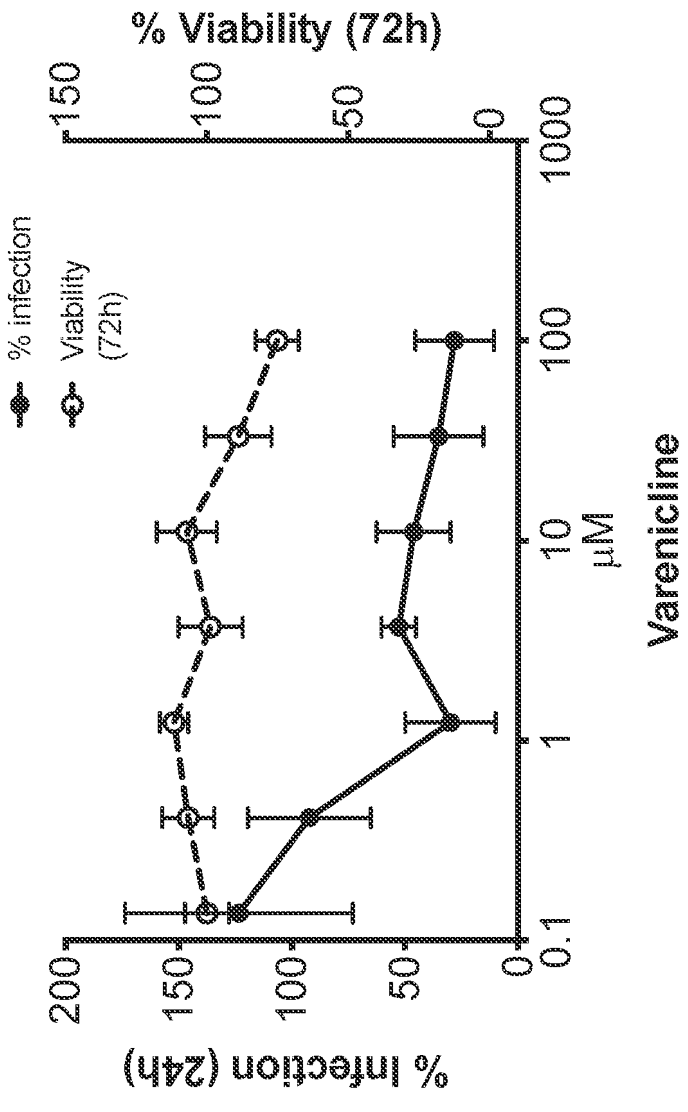
% Viability (72h)
150
100
50
0
% infection
Viability (72h)
% Infection (24h)
200
150
100
50
0
0.1
1
10
100
1000
μM
Varenicline

FIG. 3

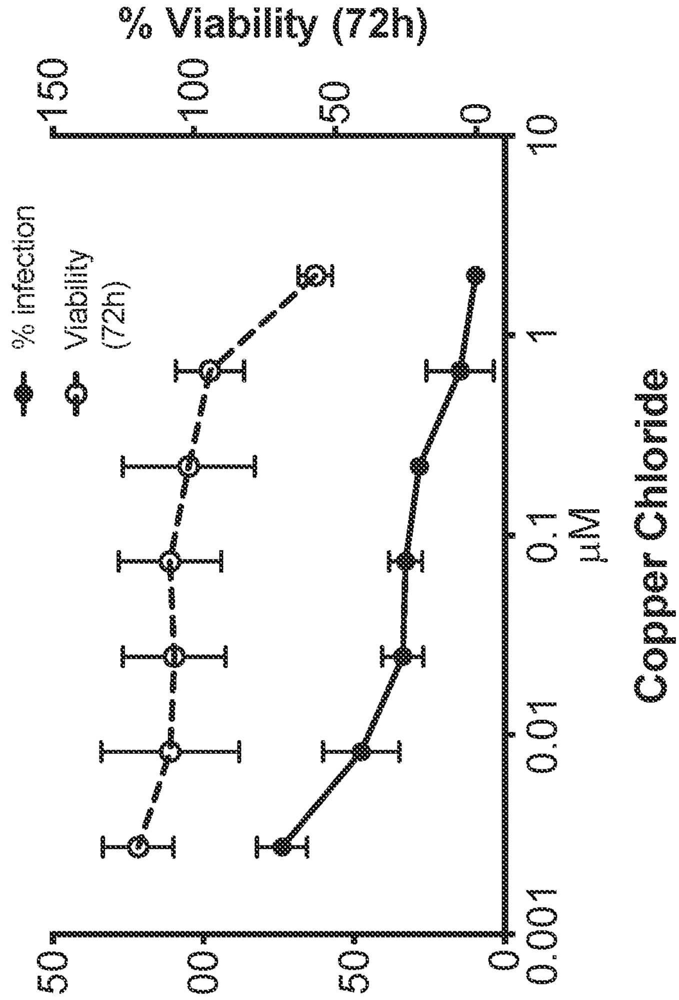
% Viability (72h)
150
100
50
0
% infection
Viability
(72h)
% Infection (24h)
150
100
50
0
0.001
0.01
0.1
1
10
µM
Copper Chloride

FIG. 4

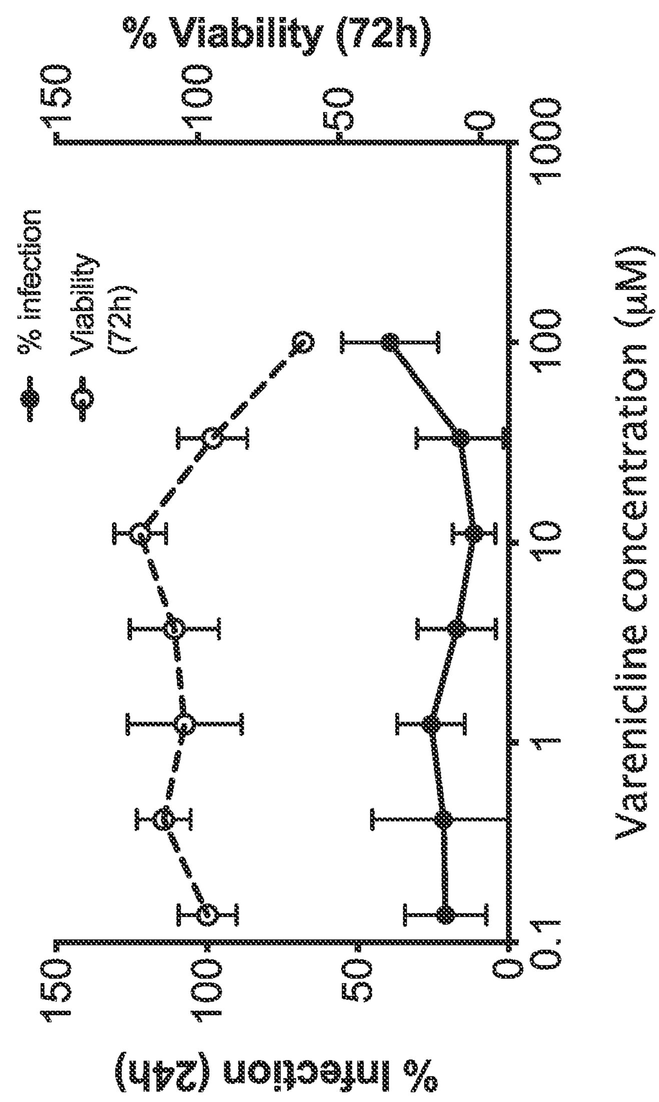


Copper Chloride 0.3 μM

FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 63016886
- US 2020281972 A1
- US 6410550 B
- US 6890927 B
- US 7265119 B
- US 9504644 B
- US 9504645 B
- US 9532944 B
- US 9597284 B
- US 10456396 B
- WO 2020014232 A
- US 7098331 B
- US 7714001 B
- US 8063068 B
- US 8067443 B
- US 8604191 B
- US 9145396 B
- US 9981949 B
- US 8633222 B
- US 10709707 B
- WO 2017177024 A
- WO 2020014217 A


### Non-patent literature cited in the description


- **CHANGEUX JEAN-PIERRE et al.** A nicotinic hypothesis for Covid-19 with preventive and therapeutic implications. *QEIOS*, 21 April 2020
- **KONSTANTINOS FARSALINOS et al.** Smoking, vaping and hospitalization for COVID-19. *QEIOS*, 03 April 2020
- **YOUSEF TIZABI et al.** Nicotine and the nicotinic cholinergic system in COVID-19. *THE FEBS JOURNAL*, 25 August 2020